(19) Europäisches Patentamt
European Patent Office
Office européen des brevets

(11) EP 2 045 603 B1

(12) **FASCICULE DE BREVET EUROPEEN**

(45) Date de publication et mention de la délivrance du brevet:
**21.03.2012 Bulletin 2012/12**

(51) Int Cl.:
***G01N 33/566*** *(2006.01)* ***G01N 33/543*** *(2006.01)*

(21) Numéro de dépôt: **09000763.4**

(22) Date de dépôt: **01.03.2001**

(54) **Biocapteurs, leur procédé d'obtention et leurs applications**

Verfahren zur Herstellung von Biosensoren und ihre Anwendung

Biosensors, uses and manufacture thereof

(84) Etats contractants désignés:
**AT BE CH CY DE DK ES FI FR GB GR IE IT LI LU MC NL PT SE TR**

(30) Priorité: **01.03.2000 FR 0002657**

(43) Date de publication de la demande:
**08.04.2009 Bulletin 2009/15**

(62) Numéro(s) de document de la (des) demande(s) initiale(s) en application de l'article 76 CBE:
**06021125.7 / 1 742 055**
**01911813.2 / 1 259 809**

(73) Titulaires:
- **Institut Pasteur**
**75015 Paris (FR)**
- **Centre National de la Recherche Scientifique**
**75016 Paris (FR)**

(72) Inventeurs:
- **Renard, Martial**
**75012 Paris (FR)**
- **Belkadi, Laurent**
**77860 Roissy en Brie (FR)**
- **England, Patrick**
**75011 Paris (FR)**
- **Bedouelle, Hugues**
**75015 Paris (FR)**

(74) Mandataire: **Leblois-Préhaud, Hélène Marthe Georgette et al**
**Cabinet Orès**
**36, rue de St Petersbourg**
**75008 Paris (FR)**

(56) Documents cités:
**WO-A-98/37226 WO-A-99/34212**
**US-A- 5 756 351**

- **SLOAN DAVID J ET AL: "Structure-based engineering of environmentally sensitive fluorophores for monitoring protein-protein interactions." PROTEIN ENGINEERING, vol. 11, no. 9, 1998, pages 819-823, XP002161420 ISSN: 0269-2139**
- **NARAZAKI RYUICHI ET AL: "Probing the cysteine 34 residue in human serum albumin using fluorescence techniques." BIOCHIMICA ET BIOPHYSICA ACTA, vol. 1338, no. 2, 1997, pages 275-281, XP000987357 ISSN: 0006-3002**
- **WU JIANHUA ET AL: "Cysteine 148 in the lactose permease of Escherichia coli is a component of a substrate binding site: 2. Site-directed fluorescence studies." BIOCHEMISTRY, vol. 33, no. 40, 1994, pages 12166-12171, XP002172185 ISSN: 0006-2960 -& JUNG HEINRICH ET AL: "Cysteine 148 in the lactose permease of Escherichia coli is a component of a substrate binding site: 1. Site-directed mutagenesis studies." BIOCHEMISTRY, vol. 33, no. 40, 1994, pages 12160-12165, XP002172186**
- **PACKARD B ET AL: "SITE-DIRECTED LABELING OF A MONOCLONAL ANTIBODY: TARGETING TO A DISULFIDE BOND" BIOCHEMISTRY,US, AMERICAN CHEMICAL SOCIETY. EASTON, PA, vol. 25, no. 12, 1986, pages 3548-3552, XP002036908 ISSN: 0006-2960**
- **POLLACK S J ET AL: "INTRODUCTION OF NUCLEOPHILES AND SPECTROSCOPIC PROBES INTO ANTIBODY COMBINING SITES" SCIENCE (WASHINGTON D C), vol. 242, no. 4881, 1988, pages 1038-1040, XP000996977 ISSN: 0036-8075**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 045 603 B1

SEQUENCE LISTING

<110> INSTITUT PASTEUR CENTRE NATIONAL DE LA RECHERCHE SCIENTIFIQUE

<120> BIOCAPTEURS, LEUR PROCEDE D'OBTENTION ET LEURS APPLICATIONS

<130> 226 cas 82

<140>
<141>

<150> FR 0002657 <151> 2000-03-01

<160> 25

<170> PatentIn Ver. 3.3

<210> 1
<211> 27
<212> DNA
<213> artificial sequence

<220> oligonucleotide
<223> pMR1-VL-T53C

<400> 1
catctgctaa gcatgttgta taataga 27

<210> 2
<211> 29
<212> DNA
<213> artificial sequence

<220> oligonucleotide
<223> pMR1-VL-S93C

<400> 2
cgaggagtac accaaaaatg ttgacagta 29

<210> 3
<211> 26
<212> DNA
<213> artificial sequence

<220> oligonucleotide
<223> pMR1-VL-T94C

<400> 3
cgtccgagga caactccaaa aatgtt 26

<210> 4
<211> 18
<212> DNA
<213> artificial sequence

<220> oligonucleotide
<223> VL-CDR1-Rev

<400> 4
agaatattgt gttcctga 18

<210> 5
<211> 18
<212> DNA
<213> artificial sequence

<220> oligonucleotide
<223> VH-CDR1-Rev

<400> 5 18
tgctgatgct cagtctgg 18

<210> 6
<211> 18
<212> DNA

<213> artificial sequence

<220> oligonucleotide
<223> VH-Seq-CDR3

<400> 6
ggtgatggaa acacagac 18

<210> 7
<211> 15
<212> DNA
<213> artificial sequence

<220> oligonucleotide
<223> pASK98-seq-His

<400> 7
cgccgcgctt aatgc 15

<210> 8
<211> 43
<212> DNA
<213> artificial sequence

<220> oligonucleotide
<223> pASK98-His6-For

<400> 8
tcgagatcaa gcggccgctg gaacaccatc accatcacca tta 43

<210> 9
<211> 43
<212> DNA
<213> artificial sequence

<220> oligonucleotide
<223> pASK98-His6-Back

<400> 9
agcttaatgg tgatggtgat ggtggtccag cggccgcttg atc 43

<210> 10
<211> 3922
<212> DNA
<213> artificial sequence

<220> plasmid
<223> pMR1

<400> 10

```
acccgacacc atcgaatggc cagatgatta attcctaatt tttgttgaca ctctatcatt 60
gatagagtta ttttaccact ccctatcagt gatagagaaa agtgaaatga atagttcgac 120
aaaaatctag ataacgaggg caaaaaatga aaaagacagc tatcgcgatt gcagtggcac 180
tggctggttt cgctaccgta gcgcaggccg aagttaaact gcaggagtca ggacctggcc 240
tggtggcgcc ctcacagagc ctgtccatca catgcaccgt ctcagggttc tcattaaccg 300
gctatggtgt aaactgggtt cgccagcctc caggaaaggg tctggagtgg ctgggaatga 360
tttggggtga tggaaacaca gactataatt cagctctcaa atccagactg agcatcagca 420
aggacaactc caagagccaa gttttcttaa aaatgaacag tctgcacact gatgacacag 480
ccaggtacta ctgtgccaga gagagagatt ataggcttga ctactggggc caagggacca 540
cggtcaccgt ctcctcaggt ggaggcggtt caggcggagg tggctctggc ggtggcggat 600
cggacatcga gctcacccag tctccagcct ccctttctgc gtctgtggga gaaactgtca 660
ccatcacatg tcgagcaagt gggaatattc acaattattt agcatggtat cagcagaaac 720
agggaaaatc tcctcagctc ctggtctatt atacaacaac cttagcagat ggtgtgccat 780
caaggttcag tggcagtgga tcaggaacac aatattctct caagatcaac agcctgcaac 840
ctgaagattt tgggagttat tactgtcaac atttttggag tactcctcgg acgttcggtg 900
gagggaccaa gctcgagatc aagcggccgc tggaacacca tcaccatcac cattaagctt 960
gacctgtgaa gtgaaaaatg gcgcacattg tgcgacattt tttttgtctg ccgtttaccg 1020
ctactgcgtc acggatctcc acgcgccctg tagcggcgca ttaagcgcgg cgggtgtggt 1080
ggttacgcgc agcgtgaccg ctacacttgc cagcgcccta gcgcccgctc ctttcgcttt 1140
cttcccttcc tttctcgcca cgttcgccgg ctttccccgt caagctctaa atcgggggct 1200
ccctttaggg ttccgattta gtgctttacg gcacctcgac cccaaaaaac ttgattaggg 1260
tgatggttca cgtagtgggc catcgccctg atagacggtt tttcgccctt tgacgttgga 1320
gtccacgttc tttaatagtg gactcttgtt ccaaactgga acaacactca accctatctc 1380
ggtctattct tttgatttat aagggatttt gccgatttcg gcctattggt taaaaaatga 1440
gctgatttaa caaaaattta acgcgaattt taacaaaata ttaacgctta caatttcagg 1500
tggcactttt cggggaaatg tgcgcggaac ccctatttgt ttatttttct aaatacattc 1560
aaatatgtat ccgctcatga gacaataacc ctgataaatg cttcaataat attgaaaaag 1620
gaagagtatg agtattcaac atttccgtgt cgcccttatt cccttttttg cggcattttg 1680
ccttcctgtt tttgctcacc cagaaacgct ggtgaaagta aaagatgctg aagatcagtt 1740
gggtgcacga gtgggttaca tcgaactgga tctcaacagc ggtaagatcc ttgagagttt 1800
tcgccccgaa gaacgttttc caatgatgag cacttttaaa gttctgctat gtggcgcggt 1860
attatcccgt attgacgccg ggcaagagca actcggtcgc cgcatacact attctcagaa 1920
tgacttggtt gagtactcac cagtcacaga aaagcatctt acggatggca tgacagtaag 1980
agaattatgc agtgctgcca taaccatgag tgataacact gcggccaact tacttctgac 2040
aacgatcgga ggaccgaagg agctaaccgc ttttttgcac aacatggggg atcatgtaac 2100
tcgccttgat cgttgggaac cggagctgaa tgaagccata ccaaacgacg agcgtgacac 2160
cacgatgcct gtagcaatgg caacaacgtt gcgcaaacta ttaactggcg aactacttac 2220
tctagcttcc cggcaacaat tgatagactg gatggaggcg gataaagttg caggaccact 2280
tctgcgctcg gcccttccgg ctggctggtt tattgctgat aaatctggag ccggtgagcg 2340
tggctctcgc ggtatcattg cagcactggg gccagatggt aagccctccc gtatcgtagt 2400
tatctacacg acggggagtc aggcaactat ggatgaacga aatagacaga tcgctgagat 2460
aggtgcctca ctgattaagc attggtagga attaatgatg tctcgtttag ataaaagtaa 2520
agtgattaac agcgcattag agctgcttaa tgaggtcgga atcgaaggtt taacaacccg 2580
taaactcgcc cagaagctag gtgtagagca gcctacattg tattggcatg taaaaaataa 2640
gcgggctttg ctcgacgcct tagccattga gatgttagat aggcaccata ctcacttttg 2700
ccctttagaa ggggaaagct ggcaagattt tttacgtaat aacgctaaaa gttttagatg 2760
tgctttacta agtcatcgcg atggagcaaa agtacattta ggtacacggc ctacagaaaa 2820
acagtatgaa actctcgaaa atcaattagc ctttttatgc caacaaggtt tttcactaga 2880
gaatgcatta tatgcactca gcgcagtggg gcattttact ttaggttgcg tattggaaga 2940
tcaagagcat caagtcgcta aagaagaaag ggaaacacct actactgata gtatgccgcc 3000
attattacga caagctatcg aattatttga tcaccaaggt gcagagccag ccttcttatt 3060
cggccttgaa ttgatcatat gcggattaga aaaacaactt aaatgtgaaa gtgggtctta 3120
aaagcagcat aacctttttc cgtgatggta acttcactag tttaaaagga tctaggtgaa 3180
gatccttttt gataatctca tgaccaaaat cccttaacgt gagttttcgt tccactgagc 3240
gtcagacccc gtagaaaaga tcaaaggatc ttcttgagat cctttttttc tgcgcgtaat 3300
ctgctgcttg caaacaaaaa aaccaccgct accagcggtg gtttgtttgc cggatcaaga 3360
gctaccaact ctttttccga aggtaactgg cttcagcaga gcgcagatac caaatactgt 3420
ccttctagtg tagccgtagt taggccacca cttcaagaac tctgtagcac cgcctacata 3480
cctcgctctg ctaatcctgt taccagtggc tgctgccagt ggcgataagt cgtgtcttac 3540
cgggttggac tcaagacgat agttaccgga taaggcgcag cggtcgggct gaacgggggg 3600
ttcgtgcaca cagcccagct tggagcgaac gacctacacc gaactgagat acctacagcg 3660
tgagctatga gaaagcgcca cgcttcccga agggagaaag gcggacaggt atccggtaag 3720
cggcagggtc ggaacaggag agcgcacgag ggagcttcca gggggaaacg cctggtatct 3780
ttatagtcct gtcgggtttc gccacctctg acttgagcgt cgatttttgt gatgctcgtc 3840
aggggggcgg agcctatgga aaaacgccag caacgcggcc tttttacggt tcctggcctt 3900
```

ttgctggcct tttgctcaca tg 3922

<210> 11
<211> 3922
<212> DNA
<213> artificial sequence

<220> plasmid
<223> pMR1 (VL-S93C)

<400> 11

acccgacacc atcgaatggc cagatgatta attcctaatt tttgttgaca ctctatcatt 60
gatagagtta ttttaccact ccctatcagt gatagagaaa agtgaaatga atagttcgac 120
aaaaatctag ataacgaggg caaaaaatga aaaagacagc tatcgcgatt gcagtggcac 180
tggctggttt cgctaccgta gcgcaggccg aagttaaact gcaggagtca ggacctggcc 240
tggtggcgcc ctcacagagc ctgtccatca catgcaccgt ctcagggttc tcattaaccg 300
gctatggtgt aaactgggtt cgccagcctc caggaaaggg tctggagtgg ctgggaatga 360
tttggggtga tggaaacaca gactataatt cagctctcaa atccagactg agcatcagca 420
aggacaactc caagagccaa gttttcttaa aaatgaacag tctgcacact gatgacacag 480
ccaggtacta ctgtgccaga gagagagatt ataggcttga ctactggggc caaggacca 540
cggtcaccgt ctcctcaggt ggaggcggtt caggcggagg tggctctggc ggtggcggat 600
cggacatcga gctcacccag tctccagcct ccctttctgc gtctgtggga gaaactgtca 660
ccatcacatg tcgagcaagt gggaatattc acaattattt agcatggtat cagcagaaac 720
agggaaaatc tcctcagctc ctggtctatt atacaacaac cttagcagat ggtgtgccat 780
caaggttcag tggcagtgga tcaggaacac aatattctct caagatcaac agcctgcaac 840
ctgaagattt tgggagttat tactgtcaac atttttggtg tactcctcgg acgttcggtg 900
gagggaccaa gctcgagatc aagcggccgc tggaacacca tcaccatcac cattaagctt 960
gacctgtgaa gtgaaaaatg gcgcacattg tgcgacattt tttttgtctg ccgtttaccg 1020
ctactgcgtc acggatctcc acgcgccctg tagcggcgca ttaagcgcgg cgggtgtggt 1080
ggttacgcgc agcgtgaccg ctacacttgc cagcgcccta gcgcccgctc ctttcgcttt 1140
cttcccttcc tttctcgcca cgttcgccgg ctttccccgt caagctctaa atcgggggct 1200
ccctttaggg ttccgattta gtgctttacg gcacctcgac cccaaaaaac ttgattaggg 1260
tgatggttca cgtagtgggc catcgccctg atagacggtt tttcgccctt tgacgttgga 1320
gtccacgttc tttaatagtg gactcttgtt ccaaactgga acaacactca accctatctc 1380
ggtctattct tttgatttat aagggatttt gccgatttcg gcctattggt taaaaaatga 1440
gctgatttaa caaaaattta acgcgaattt taacaaaata ttaacgctta caatttcagg 1500
tggcactttt cggggaaatg tgcgcggaac ccctatttgt ttatttttct aaatacattc 1560
aaatatgtat ccgctcatga gacaataacc ctgataaatg cttcaataat attgaaaaag 1620
gaagagtatg agtattcaac atttccgtgt cgcccttatt cccttttttg cggcattttg 1680
ccttcctgtt tttgctcacc cagaaacgct ggtgaaagta aaagatgctg aagatcagtt 1740
gggtgcacga gtgggttaca tcgaactgga tctcaacagc ggtaagatcc ttgagagttt 1800
tcgccccgaa gaacgttttc caatgatgag cacttttaaa gttctgctat gtggcgcggt 1860
attatcccgt attgacgccg ggcaagagca actcggtcgc cgcatacact attctcagaa 1920
tgacttggtt gagtactcac cagtcacaga aaagcatctt acggatggca tgacagtaag 1980
agaattatgc agtgctgcca taaccatgag tgataacact gcggccaact tacttctgac 2040
aacgatcgga ggaccgaagg agctaaccgc ttttttgcac aacatggggg atcatgtaac 2100
tcgccttgat cgttgggaac cggagctgaa tgaagccata ccaaacgacg agcgtgacac 2160
cacgatgcct gtagcaatgg caacaacgtt gcgcaaacta ttaactggcg aactacttac 2220
tctagcttcc cggcaacaat tgatagactg gatggaggcg gataaagttg caggaccact 2280
tctgcgctcg gcccttccgg ctggctggtt tattgctgat aaatctggag ccggtgagcg 2340
tggctctcgc ggtatcattg cagcactggg gccagatggt aagccctccc gtatcgtagt 2400
tatctacacg acggggagtc aggcaactat ggatgaacga aatagacaga tcgctgagat 2460
aggtgcctca ctgattaagc attggtagga attaatgatg tctcgtttag ataaaagtaa 2520
agtgattaac agcgcattag agctgcttaa tgaggtcgga atcgaaggtt taacaacccg 2580
taaactcgcc cagaagctag gtgtagagca gcctacattg tattggcatg taaaaaataa 2640
gcgggctttg ctcgacgcct tagccattga gatgttagat aggcaccata ctcacttttg 2700
ccctttagaa ggggaaagct ggcaagattt tttacgtaat aacgctaaaa gttttagatg 2760
tgctttacta agtcatcgcg atggagcaaa agtacattta ggtacacggc ctacagaaaa 2820
acagtatgaa actctcgaaa atcaattagc ctttttatgc caacaaggtt tttcactaga 2880
gaatgcatta tatgcactca gcgcagtggg gcattttact ttaggttgcg tattggaaga 2940
tcaagagcat caagtcgcta aagaagaaag ggaaacacct actactgata gtatgccgcc 3000
attattacga caagctatcg aattatttga tcaccaaggt gcagagccag ccttcttatt 3060
cggccttgaa ttgatcatat gcggattaga aaaacaactt aaatgtgaaa gtgggtctta 3120
aaagcagcat aacctttttc cgtgatggta acttcactag tttaaaagga tctaggtgaa 3180
gatccttttt gataatctca tgaccaaaat cccttaacgt gagttttcgt tccactgagc 3240
gtcagacccc gtagaaaaga tcaaaggatc ttcttgagat cctttttttc tgcgcgtaat 3300

```
ctgctgcttg caaacaaaaa aaccaccgct accagcggtg gtttgtttgc cggatcaaga 3360
gctaccaact ctttttccga aggtaactgg cttcagcaga gcgcagatac caaatactgt 3420
ccttctagtg tagccgtagt taggccacca cttcaagaac tctgtagcac cgcctacata 3480
cctcgctctg ctaatcctgt taccagtggc tgctgccagt ggcgataagt cgtgtcttac 3540
cgggttggac tcaagacgat agttaccgga taaggcgcag cggtcgggct gaacgggggg 3600
ttcgtgcaca cagcccagct tggagcgaac gacctacacc gaactgagat acctacagcg 3660
tgagctatga gaaagcgcca cgcttcccga agggagaaag gcggacaggt atccggtaag 3720
cggcagggtc ggaacaggag agcgcacgag ggagcttcca gggggaaacg cctggtatct 3780
ttatagtcct gtcgggtttc gccacctctg acttgagcgt cgatttttgt gatgctcgtc 3840
aggggggcgg agcctatgga aaaacgccag caacgcggcc tttttacggt tcctggcctt 3900
ttgctggcct tttgctcaca tg                                          3922
```

<210> 12
<211> 28
<212> DNA
<213> artificial sequence

<220> oligonucleotide
<223> pMR1-VL-W52C

<400> 12
gaggagtacg caaaaatgtt gacagtaa 28

<210> 13
<211> 28
<212> DNA
<213> artificial sequence

<220> oligonucleotide
<223> pMR1-VL-T52C

<400> 13
catctgctaa ggtacatgta taatagac 28

<210> 14
<211> 28
<212> DNA
<213> artificial sequence

<220> oligonucleotide
<223> pMR1-VH-Y32C

<400> 14
cagtttacac cacacccggt taatgaga 28

<210> 15
<211> 28
<212> DNA
<213> artificial sequence

<220> oligonucleotide
<223> pMR1-VH-T30C

<400> 15
acaccatagc cgcataatga gaaccctg 28

<210> 16
<211> 31

<212> DNA
<213> artificial sequence

<220> oligonucleotide
<223> pMR1-VL-D56C

<400> 16
cttgatggca caccgcatgc taaggttgtt g 31

<210> 17
<211> 31
<212> DNA
<213> artificial sequence

<220> oligonucleotide
<223> pMRl-VL-N31C

<400> 17
catgctaaat aacagtggat attcccactt g 31

<210> 18
<211> 33
<212> DNA
<213> artificial sequence

<220> oligonucleotide
<223> pMR1-VH-S28C

<400> 18
acaccatagc cagttaagca gaaccctgag acg 33

<210> 19
<211> 32
<212> DNA
<213> artificial sequence

<220> oligonucleotide
<223> pMR1-VH-N56C

<400> 19
ttatagtctg tgcacccatc accccaaatc at 32

<210> 20
<211> 31
<212> DNA
<213> artificial sequence

<220> oligonucleotide
<223> pMR1-VL-H30C

<400> 20
atggtacgat ttattaaaca ttataagggt g 31

<210> 21
<211> 34
<212> DNA
<213> artificial sequence

<220> oligonucleotide
<223> pMR1-VL-Y49C

<400> 21
agacgattcc aacaacatat acactggtcc tcga 34

<210> 22
<211> 35
<212> DNA
<213> artificial sequence

<220> oligonucleotide
<223> pMR1-VL-Y50C

<400> 22
gattccaaca acatatctgg tcctcgactc ctcta 35

<210> 23
<211> 36
<212> DNA
<213> artificial sequence

<220> oligonucleotide
<223> pMR1-VH-R99C

<400> 23
tgacacggtc tctcacacta atatccgaac tgatga 36

<210> 24
<211> 28
<212> DNA
<213> artificial sequence

<220> oligonucleotide
<223> pMR1-VH-G31C

<400> 24
caagagtaat tggacaatac cacatttg 28

<210> 25
<211> 32
<212> DNA
<213> artificial sequence

<220> oligonucleotide
<223> pMR1-VH-G53C

<400> 25
acccttacta aacacaacta cctttgtgtc tg 32

## Description

La présente invention est relative à des biocapteurs, à leur procédé d'obtention et à leurs applications notamment pour la détection, le dosage ou la localisation, en immunofluorescence directe, d'un ligand tel qu'un antigène ou un haptène, dans une population hétérogène.

Un biocapteur est une macromolécule biologique bifonctionnelle :

d'une part, il est capable de fixer spécifiquement son ligand dans un mélange, d'autre part, il transduit l'événement de fixation en un signal mesurable directement et
instantanément : au moyen d'un simple système de diodes, d'un spectrofluorimètre,
d'un microscope à fluorescence, d'un microscope confocal, ou de tout autre appareil,
avec pour conséquence l'immédiateté de la détection ainsi effectuée.

Si les « puces » à ADN permettant la détection des séquences d'acides nucléiques existent déjà, une solution générale pour la construction de biocapteurs et de puces à base de protéines, permettant la détection d'autres ligands n'existe pas et reste donc à mettre au point.

Plusieurs approches en ce sens ont été tentées :

- Un fluorophore sensible à son environnement électronique a été couplé à une protéine par l'intermédiaire de la fonction thiol d'une unique cystéine libre, naturellement présente dans la protéine ou introduite dans la protéine par mutagénèse dirigée. La cystéine est introduite, soit dans le site de liaison du ligand ou au voisinage immédiat de ce site (protéine GB1 : Sloan et al., 1998 ; protéine GBP : WO 99/34212), soit à distance de ce site (protéine MBP : Gilardi et al., 1994 ; Marvin et al., 1997 ; protéine GBP : Marvin et al., 1998 ; Tolosa et al., 1999 ; Albumine : Narazaki et al. ; protéine transmembranaire sensible aux changements de surface de la cellule : Brevet US 5,756,351). La fixation du ligand modifie alors l'environnement électronique du fluorophore et le changement des propriétés du biocapteur est ainsi directement détectable par spectrofluorimétrie. Par exemple, Sloan et al. (1998) ont notamment montré que le fluorophore doit être positionné dans une région située à l'interface protéine-protéine et ils ont étudié le couplage d'un fluorophore dans une région de ce type, pour le complexe domaine GB1-Fc : dans ce contexte, la formation du complexe est analysée ; les meilleurs résultats sont obtenus, lorsque le fluorophore est couplé au niveau d'un acide aminé impliqué dans la formation du complexe.

Toutefois cette approche ne concerne que des protéines très particulières, généralement sans résidu de cystéine libre et sans résidus de cystéine impliqués dans des ponts disulfures. Par ailleurs, l'utilisation des biocapteurs précités est limitée à la détection de quelques ligands particuliers (maltose, glucose et fragment Fc d'anticorps). En effet, dans cette approche, la difficulté principale pour la création de biocapteurs, à partir d'une protéine qui contient des ponts disulfures vient de ce que les étapes du couplage du fluorophore à un résidu Cys peuvent résulter en une attaque de ponts disulfures essentiels pour la structure et la stabilité et une inactivation de la molécule. Parmi ces protéines, les anticorps représentent la classe de protéines naturellement dédiées à la fixation spécifique de ligands protéiques, peptidiques, polysaccharidiques ou hapténiques (antigènes) avec une grande affinité. Cependant, les molécules d'anticorps possèdent des résidus Cys formant des ponts disulfures intra- et inter-chaînes. En particulier, les fragments Fv d'anticorps comportent deux ponts disulfures, un dans chacun des domaines VH et VL, essentiels pour la stabilité du fragment Fv ou des fragments à chaîne unique scFv (Glockshuber et al., 1992).

- un biocapteur a été dérivé d'un anticorps qui fixe le 2,4-dinitrophénol par l'approche suivante. Un groupement thiol a été introduit au voisinage du site de fixation du ligand au moyen d'un marqueur d'affinité qui combinait, en une même molécule, un groupement 2,4-dinitrophénol reconnu spécifiquement par l'anticorps, un lien thiophényl ou disulfure, et un groupement réactif aldéhyde ou alpha-bromocétone. Le marqueur d'affinité était capable de se lier au site de fixation de l'anticorps par l'intermédiaire du groupement 2,4-dinitrophénol, puis de se coupler à l'anticorps par réaction au hasard de son groupement aldéhyde ou alpha-bromocétone avec les chaînes latérales des résidus de Lys, His ou Tyr situés à proximité. Le traitement de l'anticorps couplé, avec du dithiothréitol, libérait un groupement SH qui pouvait à son tour être couplé à un fluorophore (Pollack et al. 1988).

Cette méthode présente l'inconvénient d'être aléatoire et non généralisable et de ne s'appliquer qu'à des anticorps dirigés contre des haptènes car elle dépend de l'existence de résidus Lys, His ou Tyr à bonne distance du site de fixation de l'anticorps, et de la possibilité de synthétiser un marqueur d'affinité tripartite comme décrit ci-dessus.

Les Inventeurs se sont donnés pour but de permettre l'obtention de biocapteurs à base de protéines par une méthode rationnelle, générale et généralisable, apte à identifier les résidus d'acide aminé d'un récepteur protéique qui peuvent être substitués en résidus cystéine. De tels biocapteurs répondent mieux aux besoins de la pratique, notamment en ce qu'ils permettent le couplage d'un fluorophore à une position induisant un changement de l'environnement structural du fluorophore lors de la fixation du ligand, que la structure cristalline du complexe entre le récepteur et le ligand soit connue ou non.

De tels biocapteurs peuvent être construits à partir de tout type de protéines, notamment des protéines possédant des résidus cystéines importants pour leur structure ou leur stabilité, et constituent de nouveaux outils de détection, de dosage et de localisation d'une grande variété de ligands dans une population hétérogène de molécules

La présente invention a pour objet un biocapteur, caractérisé en ce qu'il est constitué par au moins un fragment de récepteur de nature protéique possédant un ou plusieurs ponts disulfures indispensables à son activité ou au maintien de sa structure et apte à se lier à un ligand convenable par l'intermédiaire d'un site de liaison, lequel fragment comprenant un fluorophore couplé à l'atome en position γ d'au moins l'un de ses résidus d'acide aminé naturellement présent sous forme de résidu Cys ou substitué en résidu Cys lequel résidu d'acide aminé, situé au voisinage des résidus du site de liaison le long de la séquence du récepteur, est sélectionné dans le groupe constitué par les résidus qui sont en contact direct avec le ligand, ceux qui sont en contact par l'intermédiaire d'une molécule d'eau, et ceux dont la surface accessible au solvant est modifiée par la fixation du ligand, , et (ii) et lequel biocapteur est susceptible d'être obtenu par un procédé comprenant les étapes suivantes :

(a) identification des résidus d'acide aminé du site de liaison du récepteur par mutagénèse de l'ensemble ou d'un sous-ensemble des résidus d'acide aminé du récepteur, et détermination des variations des paramètres d'interaction avec le ligand ($K_D$, $k_{on}$, $k_{off}$) qui sont dues à chaque mutation ou à des groupes limités de mutations ;
(b) sélection des résidus Cys ou des résidus à muter en cystéine parmi les résidus du récepteur qui sont situés au voisinage des résidus du site de liaison le long de la séquence du récepteur ;
(c) mutation dirigée d'au moins l'un des résidus d'acide aminé sélectionné en (b), en un résidu Cys, dans le cas où ledit résidu n'est pas naturellement un résidu Cys ;
(d) réduction ménagée du récepteur obtenu en (b) ou en (c) ; et
(e) couplage de l'atome Sγ d'au moins un résidu Cys obtenu en (b) ou en (c) avec un fluorophore.

La réduction ménagée (étape (d)) permet d'améliorer significativement le rendement de couplage (étape (e)).

Les résidus Cys qui ne sont pas essentiels, par exemple ceux qui sont situés loin du site actif, peuvent être changés en d'autres résidus par mutagénèse dirigée, notamment en résidus Ser ou Ala, pour éviter des couplages parasites avec les fluorophores.On entend par récepteur, au sens de la présente invention, une macromolécule protéique possédant un site actif, capable de fixer un ligand, notamment un anticorps, un récepteur hormonal ou bactérien, une protéine affine, une protéine de transport ou un récepteur viral ou encore tout polypeptide ayant une affinité spécifique pour un ligand.

On entend par ligand, au sens de la présente invention, toute molécule apte à se lier audit récepteur par l'intermédiaire dudit site actif, notamment : un antigène protéique, peptidique ou hapténique, tel qu'un antigène bactérien, ou une hormone, une cytokine, une interleukine, le TNF (*Tumor Necrosis Factor*), un facteur de croissance, une protéine virale ou une séquence peptidique ou nucléotidique.

Au sens de la présente invention, on entend par site actif du récepteur ou du fragment de récepteur l'ensemble des résidus qui participent à la liaison du ligand. Ce site actif est encore appelé site de liaison ou paratope.

Au sens de la présente invention, le terme voisinage s'entend au sens défini par la théorie mathématique des espaces topologiques ; les résidus du récepteur, au voisinage du site actif sont les résidus qui sont en contact direct avec le ligand, ceux qui sont en contact par l'intermédiaire d'une molécule d'eau, et ceux dont la surface accessible au solvant (ASA ; Creighton, 1993) est modifiée par la fixation du ligand. L'utilisation de sphères de rayon croissant, de 1,4 à 30 Å maximum, de préférence, de 1,4 à 2,9 Å, c'est-à-dire plus élevé que celui d'une molécule d'eau (1,4 Å), par exemple 1,4 ; 1,7 ; 2,0 ; 2,3 ; 2,6 et 2,9 Å, pour calculer la surface accessible au solvant, permet de définir un voisinage de plus en plus large du site de liaison du récepteur, et ainsi d'augmenter l'ensemble des sites potentiels de couplage du fluorophore en tenant compte du volume important d'un fluorophore par rapport à celui d'une molécule d'eau. La mutation en cystéine ne doit pas être fortement délétère pour l'interaction avec le ligand, si l'on veut que le récepteur marqué conserve une bonne affinité pour le ligand.

Au sens de la présente invention, on entend par fluorophore, toute molécule dont la fluorescence est sensible à son micro-environnement et qui peut-être couplée à un résidu Cys.

Conformément à l'invention, le fluorophore est notamment sélectionné dans le groupe constitué par : le *N*-((2-iodoacetoxy)ethyl)-*N*-methyl)amino-7-nitrobenz-2-oxa-1,3diazole (IANBD), le 4-chloro-7-nitrobenz-2-oxa-1,3-diazole (CNBD), l'acrylodan, la 5-iodoacétamidofluorescéine (5-IAF), ou un fluorophore possédant une chaîne aliphatique de 1 à 6 atomes de carbone.

Selon un mode de réalisation avantageux dudit biocapteur, le biocapteur est sous forme soluble ou est immobilisé sur un support solide convenable en matière plastique ou en verre.

Selon encore un autre mode de réalisation avantageux dudit biocapteur, ledit support solide est une microplaque ou une fibre optique.

Parmi les procédés d'immobilisation, on peut citer ceux décrits dans Piervincenzi et al., 1998 ; Yoshioka et al., 1991 ; Turkova, 1999 ; Saleemuddin, 1999 ; Weetall, 1993 ; Sara et al., 1996.

Lorsque le support est une fibre optique, de tels biocapteurs peuvent être implantés *in situ*, par exemple dans une veine d'un patient, d'un animal ou dans une cellule individuelle, pour doser le ligand *in vivo*, de façon continue, et ainsi suivre sa cinétique d'apparition et de disparition.

La présente invention a également pour objet une puce à base de protéines, caractérisée en ce qu'elle est constituée par un support solide sur lequel est immobilisé au moins un biocapteur selon l'invention.

De telles puces à base de protéines comprennent un support solide, par exemple une microplaque, sur laquelle les molécules de différents biocapteurs sont avantageusement immobilisées en matrice et incluses dans des circuits micro-fluidiques. De tels biocapteurs permettent de détecter et de doser simultanément, et de façon instantanée, un grand nombre de ligands dans un échantillon, par exemple un échantillon de fluide corporel.

Les biocapteurs selon l'invention présentent un certain nombre d'avantages :

- le résidu du récepteur sur lequel le couplage du fluorophore est réalisé, est prédéterminé et est un résidu Cys situé au voisinage du site actif du récepteur.
- le couplage du fluorophore sur des résidus cystéine prédéterminés est réalisé dans des conditions où il n'attaque pas les ponts disulfures éventuellement présents sur le récepteur.

La présente Demande divulgue un procédé de préparation de biocapteurs tels que définis ci-dessus, comprenant les étapes suivantes :

(a) sélection de résidus du récepteur par recherche des résidus qui sont, dans le complexe récepteur-ligand, (i) en contact direct avec le ligand, ou (ii) en contact par l'intermédiaire d'une molécule d'eau, ou (iii) dont la surface accessible au solvant (ASA) est modifiée par la fixation du ligand, lorsqu'on utilise des sphères de rayon croissant de 1,4 à 30 Å, de préférence de 1,4 à 2,9 Å pour la molécule dudit solvant ;

(b) calcul de l'accessibilité au solvant (ASA), pour le récepteur libre, des atomes en position γ et éventuellement en position δ pour chaque résidu d'acide aminé sélectionné en (a), en utilisant une sphère de rayon 1,4 Å (correspondant à une molécule d'eau), et sélection des résidus dont l'atome en position γ ou l'atome en position δ est accessible au solvant ; l'accessibilité au solvant d'un atome en position δ est informative sur celle du Sγ d'un résidu Cys correspondant. De manière habituelle, le seuil d'accessibilité est fixé au minimum à 2 %, de préférence, à 20 -25 % et plus favorablement, supérieur à 25 % ;

(c) mutation dirigée d'au moins l'un des résidus, sélectionné en (b) en un résidu Cys, dans le cas où ledit résidu n'est pas naturellement un résidu Cys, et

(d) couplage de l'atome Sγ d'au moins un résidu Cys obtenu en (b) ou (c) avec un fluorophore. Dans ce cas, l'atome Sγ du résidu Cys mutant ou non qui est exposé au solvant est la cible de la molécule de fluorophore. On considère que le Sγ du résidu Cys mutant ou non se superpose avec l'atome en position γ du résidu de type sauvage. Cet atome en position γ doit donc être exposé au solvant dans la structure du récepteur sauvage. Lorsqu'un atome est présent en position δ dans le résidu de type sauvage, il peut masquer l'atome en position γ vis-à-vis du solvant alors que ce masquage n'existera plus après son changement en Cys.

A l'étape (b), les valeurs d'ASA d'un résidu $X_i$ en position i de la séquence du récepteur, peuvent être exprimées sous forme de pourcentages des valeurs d'ASAs correspondantes dans un tripeptide Gly-$X_i$-Gly, qui adopterait la même conformation que le tripeptide $X_{i-1}$-$X_i$-$X_{i+1}$ dans la structure du récepteur.

Les résidus retenus à l'étape (b) sont divisés en trois classes :

- la première classe contient les résidus dont l'atome en position γ est accessible au solvant dans la structure du récepteur libre, et qui sont en contact avec le ligand soit directement, soit par l'intermédiaire d'une molécule d'eau ;
- la deuxième classe contient les résidus dont l'atome en position γ est accessible et qui ne sont pas en contact direct avec le ligand ;
- la troisième classe contient les résidus dont l'atome en position δ est accessible, mais pas l'atome en position γ.

Lorsque la structure cristalline de l'un des partenaires du couple récepteur/ligand ou de leur complexe n'est pas connue ou pas disponible, dans ce cas, ledit procédé comprend préalablement à l'étape (a) telle que définie ci-dessus, une étape de modélisation de la molécule dont la structure n'est pas connue ou pas disponible : récepteur ou fragment actif de ce récepteur (par exemple un fragment Fv d'anticorps), son ligand (antigène), ou leur complexe (récepteur-ligand).

Ladite étape de modélisation peut être mise en oeuvre par une méthode assistée par ordinateur ; on peut citer par exemple les méthodes décrites dans Sternberg, 1996 ; Rees et al., 1996.

La présente Demande divulgue également un procédé de préparation de biocapteurs qui comprend les étapes suivantes :

($a_1$) identification du site actif du récepteur par mutagénèse de l'ensemble ou d'un sous-ensemble des résidus du récepteur, et détermination des variations des paramètres d'interaction avec le ligand ($K_D$, $k_{on}$, $k_{off}$) qui sont dues à chaque mutation ou à des groupes limités de mutations ;
($b_1$) sélection des résidus Cys ou des résidus à muter en cystéine parmi les résidus du récepteur qui sont situés au voisinage des résidus du site actif le long de la séquence ;
($c_1$) mutation dirigée d'au moins l'un des résidus sélectionné en ($b_1$), en un résidu Cys dans le cas où ledit résidu n'est pas naturellement un résidu Cys ; et
($d_1$) couplage de l'atome Sγ d'au moins un résidu Cys obtenu en ($b_1$) ou en ($c_1$) avec un fluorophore.

Par exemple, les séquences des boucles hypervariables de l'anticorps mAbD1.3, dénommées CDRs (*Complementary Determining Regions* ou régions qui déterminent la complémentarité anticorps-antigène), peuvent être définies par des comparaisons de séquences (England et al., 1999). Le site actif de mAbD1.3 pour son interaction avec le lysozyme a été caractérisé par des changements des résidus des CDRs, principalement en Ala (Dall'Acqua et al., 1996, 1998 ; England et al., 1997, 1999 ; Ito et al., 1993, 1995 ; Hawkins et al., 1993 ; Ysern et al., 1994 ; Goldbaum et al., 1996). Les changements de résidus des chaînes légères (L) L-H30A, L-Y32A, L-Y49A, L-Y50A et L-Y92A augmentent la constante de dissociation entre le fragment Fv ou scFv de mAbD1.3 et le lysozyme, tandis que les changements L-I29T, L-T51A, L-T52K, L-T53A, L-S93A et L-T97S l'affectent très peu ou pas du tout. De même, les changements de résidus des chaînes lourdes (H) H-F27N, H-Y32A, H-W52A, H-D54A, H-D100A, H-Y101A, H-R102K ou H-R102M augmentent la constante de dissociation tandis que H-T30A, H-N56A, H-D58A et H-R99A l'affectent peu ou pas du tout.

Par conséquent, les résidus L-Thr51, L-Thr52, L-Thr53, L-Ser93, L-Thr94, H-Thr30, H-Asn56, H-Asp58 et H-Arg99 qui sont polaires et donc vraisemblablement exposés à la surface de l'anticorps mAbD1.3 et qui sont proches des résidus du site actif dans la séquence, constitueraient des sites potentiels de couplage suivant des critères purement fonctionnels. On retrouve bien certains sites potentiels communs lorsqu'on utilise des critères basés sur la structure (points (a) et (b) du procédé général, ci-dessus) ou sur la fonction (points ($a_1$) et ($b_1$) de la variante dudit procédé, ci-dessus), par exemple L-Thr52, L-Thr53, L-Ser93, H-Thr30, H-Asn56, H-Asp58 et H-Arg99.

Préalablement à l'étape (a) ou à l'étape ($a_1$), les résidus Cys non essentiels du récepteur peuvent être substitués en résidus Ser ou Ala par mutagénèse dirigée.

A l'étape (d) ou à l'étape ($d_1$), ledit fluorophore peut être sélectionné dans le groupe constitué par le *N*-((2-iodoacetoxy)ethyl)-*N*-methyl)amino-7-nitrobenz-2-oxa-1,3diazole (IANBD), le 4-chloro-7-nitrobenz-2-oxa-1,3-diazole (CNBD), l'acrylodan, la 5-iodoacétamidofluorescéine ou un fluorophore possédant une chaîne aliphatique de 1 à 6 atomes de carbone.Préalablement à l'étape (d) ou à l'étape ($d_1$), le récepteur muté obtenu à l'étape (c) ou à l'étape ($c_1$) peut être soumis à une réduction ménagée ; en effet, le rendement de couplage peut-être significativement amélioré, lorsque l'on ajoute cette étape.

Après l'étape (d) ou l'étape ($d_1$), il peut comprendre une étape supplémentaire (e) ou ($e_1$), de purification du biocapteur, par exemple sur colonne d'exclusion ou d'affinité, en particulier une colonne d'ions nickel immobilisés, dans le cas où le fragment de récepteur ou d'anticorps comprend une extension de résidus His.

Après l'étape (e) ou l'étape ($e_1$), il peut comprendre une étape supplémentaire de mesure de la constante d'équilibre ($K_D$ ou $K'_D$), ou des constantes de vitesse de dissociation ($k_{off}$) et d'association ($k_{on}$) entre le récepteur et le ligand.

La constante d'équilibre est dénommée $K_D$ lorsque le complexe est en solution et $K'_D$ lorsque l'un des partenaires du complexe est immobilisé.

Ladite constante peut par exemple être déterminée à partir de mesures de fluorescence en intensité ou en anisotropie. L'ensemble des données obtenues ($K_D$, $K'_D$, $k_{off}$, $k_{on}$, amplitude du changement de fluorescence lors de la fixation du ligand, déplacement du maximum d'émission) permettent d'affiner la sélection des résidus du récepteur les plus appropriés pour le couplage du fluorophore.

Après les étapes (d) ou ($d_1$), ou les étapes (e) ou ($e_1$), il peut comprendre une étape supplémentaire d'immobilisation du biocapteur sur un support solide convenable tel que cité précédemment.

La présente invention a également pour objet l'utilisation desdits biocapteurs, seuls ou en mélange, pour des applications de détection, de dosage et de localisation de ligands.

Les biocapteurs selon l'invention permettent par exemple de détecter, doser ou localiser un antigène ou un haptène dans une population hétérogène de molécules, de façon instantanée.

Dans le domaine de la santé, lesdits biocapteurs trouvent des applications :

- pour suivre la progression ou la régression d'une maladie, en réponse à un traitement ;

- pour doser un agent infectieux (bactérie, virus), un agent pathogène (cellule tumorale), une macromolécule (hormone, cytokine) ou un haptène dans un fluide corporel (par exemple sang, sperme, etc) ;
- pour déterminer le sérotype d'un agent infectieux ;
- pour détecter un marqueur cellulaire et déterminer sa localisation ;
- pour le tri de cellules, sur la base de la présence d'un marqueur de surface ;
- pour le tri de molécules ;
- pour quantifier un composant intra-cellulaire ou extracellulaire, suivre sa cinétique d'apparition et de disparition, et pour suivre sa localisation ;
- pour déterminer la demi-vie d'une molécule (dépendant de son instabilité, de son métabolisme, ou de son élimination), sa diffusion, et sa concentration tissulaire ;
- pour suivre l'effet d'une molécule chimique sur un composant cellulaire (criblage de banques, notamment de banques protéiques).

Dans le domaine de l'environnement, de l'industrie et de la répression des fraudes, lesdits biocapteurs trouvent également des applications :

- pour détecter et doser un polluant, et suivre son élimination, par des processus naturels ou de biorémédiation ;
- pour doser un composant actif dans une préparation en cours de fabrication ou de commercialisation ;
- pour suivre la cinétique d'une réaction de synthèse chimique. Lesdits biocapteurs trouvent également application dans la fabrication de puces à base de protéines.

La présente invention a également pour objet des réactifs de détection, de dosage et/ou de localisation de ligands, caractérisés en ce qu'ils comprennent au moins un biocapteur tel que défini ci-dessus.

La présente Demande divulgue un procédé de détection, de dosage ou de localisation d'un ligand dans un échantillon hétérogène comprenant la mise en contact dudit échantillon hétérogène avec au moins un réactif selon l'invention.

La présente Demande divulgue une trousse de détection, de dosage et/ou de localisation de ligands incluant au moins un réactif selon l'invention.

La présente Demande divulgue une trousse de criblage d'inhibiteurs de l'interaction ligand/récepteur incluant au moins un biocapteur selon l'invention.

La présente Demande divulgue l'utilisation du plasmide pMR1 de séquence SEQ ID NO:10, déposé auprès de la Collection Nationale de Culture de Microorganismes (CNCM), sous le numéro 1-2386, en date du 29 février 2000, pour la préparation d'un biocapteur conforme à l'invention.

La présente Demande divulgue l'utilisation du plasmide pMR1(VL-S93C) de séquence SEQ ID NO:11, déposé auprès de la Collection Nationale de Culture de Microorganismes (CNCM), sous le numéro 1-2387, en date du 29 février 2000, pour la préparation d'un biocapteur conforme à l'invention.

La présente Demande divulgue le plasmide pMR1 de séquence SEQ ID NO:10, déposé auprès de la Collection Nationale de Culture de Microorganismes (CNCM), sous le numéro I-2386, en date du 29 février 2000.

La présente Demande divulgue le plasmide pMR1(VL-S93C) de séquence SEQ ID NO:11, déposé auprès de la Collection Nationale de Culture de Microorganismes (CNCM), sous le numéro I-2387, en date du 29 février 2000.

Outre les dispositions qui précèdent, la demande comprend encore d'autres dispositions, qui ressortiront de la description qui va suivre, qui se réfère à des exemples de mise en oeuvre du procédé objet de la présente invention ainsi qu'aux dessins annexés, dans lesquels :

- La figure 1 illustre les critères de détermination des résidus du fragment Fv de l'anticorps dénommé mAbD1.3 au voisinage du site actif de liaison au lysozyme, utilisables pour le couplage du fluorophore. Les **colonnes 1 et 2** indiquent les résidus du fragment Fv de l'anticorps dénommé mAbD1.3 qui sont en contact direct avec le lysozyme, soit au moins par leur chaîne latérale **(d)**, soit par leur chaîne peptidique (b) ; ceux qui sont en contact par l'intermédiaire d'une molécule d'eau (i), et ceux dont la surface accessible au solvant (ASA) est modifiée par la fixation du lysozyme lorsqu'on utilise des sphères de rayon 1,4 ; 1,7 ; 2,0 ; 2,3 ; 2,6 ou 2,9 Å pour la molécule de solvant. **Les colonnes 3 et 4** indiquent les valeurs d'ASA, dans le Fv libre (en utilisant R = 1,4 Å), des atomes en position γ et éventuellement δ de chacun des résidus $X_i$ sélectionnés, en position i de la séquence du Fv, sous forme de pourcentages des ASAs correspondantes dans un tripeptide Gly-$X_i$-Gly qui adopterait la même conformation que le tripeptide $X_{i-1}$-$X_i$-$X_{i+1}$ dans la structure du Fv. **Les colonnes 5, 6 et 7** indiquent l'ASA dans la structure du complexe natif, des groupements en positions γ, δ et ε dans la chaîne latérale initiale. **Les colonnes 8 et 9** indiquent les mutations de ces résidus connues dans mAbD1.3 et leurs effets, sous forme d'une variation ΔΔG d'énergie d'interaction avec le lysozyme. **La colonne 10** résume le classement des résidus par ordre de priorité décroissante ; (+) correspond aux résidus dont l'atome en position γ est accessible au solvant dans la structure du Fv libre, et qui sont en contact avec le lysozyme soit directement, soit par l'intermédiaire d'une molécule d'eau dans la structure du complexe, (+/-) correspond aux résidus dont l'atome en position $\gamma$ est accessible et qui ne sont pas en contact direct avec le lysozyme et (?) correspond aux résidus dont l'atome en position $\delta$ est accessible, mais pas l'atome en position $\gamma$.

- La figure 2 illustre l'optimisation de la production de Fv de mAbD1.3 recombinant (scFv-His6), à l'aide de vecteurs d'expression procaryotes (pMR1 et pMR5). Le contenu périplasmique de souches HB2151 et BL21 (DE3) de E. *Coli* transformées respectivement par les plasmides pMR5 et pMR1, induites par l'IPTG (BL21 (DE3)) ou l'anhydrotétracycline (HB2151) et cultivées en faisant varier la température, le temps d'induction et la concentration de l'inducteur, a été mesuré par ELISA à l'aide d'un anticorps anti-His5. Les résultats sont exprimés en unités arbitraires.
- La figure 3 illustre l'analyse par électrophorèse en gel de polyacrylamide-SDS et coloration au bleu de Coomassie des fractions de la purification du scFv-His6(VL-S93C) mutant, sur colonne d'ions nickel. **EP** : extrait périplasmique non-purifié, **NR** : fractions non retenues, **M** : marqueur de masse moléculaire, $L_{20}$ et $L_{40}$ : fractions de lavage à 20 et 40 mM d'imidazole, **EL** : fractions d'élution.
- Les figures 4 et 5 illustrent l'oligomérisation non-covalente du scFv-His6 de type sauvage et du mutant VL-S93C, analysée par chromatographie d'exclusion de taille. Les scFv-His6 purifiés ont été injectés à une concentration de 50 $\mu$g/ml pour le type sauvage et pour le mutant, dans un volume de **200** $\mu$L, en haut d'une colonne Superdex$^R$ 75 HR 10/30 (Pharmacia). Le chromatogramme a été développé à 25°C, à pH=7,5 pour le type sauvage et à pH=7,0 pour le mutant.
- La figure 6 représente les structures chimiques du CNBD, de l'IANBD, de la 5-iodoacétamidofluorescéine (5-IAF) et de l'acrylodan.
- La figure 7 illustre la proportionnalité entre intensité de fluorescence (excitation à 469 nm, émission à 535 nm) et concentration en protéine dans les fractions d'élution issues de la séparation entre conjugué et fluorophore libre.
- La figure 8 illustre l'émission de fluorescence par des dérivés du scFv-His6, pour une excitation à 469 nm, en l'absence de lysozyme. Les espèces suivantes ont été analysées : scFv-His6 de type sauvage et scFv-His6(VL-S93C) après couplages avec l'IANBD et séparations ; scFv-His6(VL-S93C) avant couplage.
- La figure 9 montre l'évolution du rendement de couplage (proportion de scFv-His6 mutants liés à un fluorophore, ici l'IANBD) et du rendement en matériel lors de la préparation du biocapteur, en fonction de la concentration en 2-mercaptoéthanol utilisée pour la réduction ménagée.
- La figure 10 montre le spectre d'absorption du dérivé fluorescent scFv-His6(VL-S93ANBD), enregistré pour des longueurs d'onde de 250 à 550 nm. La hauteur du pic à 278 nm, correspondant à la protéine, et celle du pic à 500 nm, correspondant à l'IANBD couplé, ont permis de calculer le rendement de couplage (ici 83%).
- La figure 11 illustre l'émission de fluorescence par le dérivé scFv-His6(VL-S93ANBD) pour une excitation à 469 nm exprimée en unité abritraire (v.a.). Les spectres ont été enregistrés en l'absence de lysozyme, ou 1 minute après addition de lysozyme à 100 nM et 1 $\mu$M (concentrations finales) et brève homogénéisation. La même exaltation maximale de fluorescence (+27%) est observée pour trois préparations différentes de ce même dérivé pour lesquelles différentes méthodes de couplage (avec ou sans traitement réducteur préalable) ont été utilisées, et différents rendements de couplage ont été obtenus.
- La figure 12 illustre l'émission de fluorescence à 535 nm par le conjugué scFv-His6(VL-S93ANBD) pour une excitation à 480 nm, exprimée en unité arbitraire (v.a.). Les intensités de fluorescence ont été enregistrées en l'absence ou en présence de concentrations variables de lysozyme (10 nM à 2 $\mu$M) dans un tampon 50 mM Tris-HCl, 150 mM NaCl, pH=7,5. Dans ce tampon, l'intensité de fluorescence du conjugué scFv-His6(VL-S93ANBD) est proportionnelle à la concentration de lysozyme jusqu'à 400 nM et augmentée de 91 % à saturation. La courbe obtenue permet de titrer directement le lysozyme entre 10 et 400 nM.
- La figure 13 illustre les rendements de couplage et les propriétés de fluorescence des biocapteurs. La colonne 1 indique les résidus du fragment Fv de l'anticorps dénommé mAbD1.3 qui ont été mutés en résidus Cys ; ces résidus ont été choisis en fonction des données structurales exposées dans la figure 1. La colonne 2 indique le pourcentage de molécules du scFv-His6 mutant couplées à l'IANB, par comparaison au scFv-His6 de type sauvage (wt). La colonne 3 indique la variation de fluorescence à 535 nm entre les formes libres et complexées au lysozyme des conjugués scFv-Cys-ANBD, mesurée dans un tampon 20 mM Tris-HCl, 500 mM NaCl, 160 mM imidazole, pH=7,9. Des exaltations importantes de fluorescence sont obtenues pour plus de la moitié des positions de couplage.

Il doit être bien entendu, toutefois, que ces exemples sont donnés uniquement à titre d'illustration de l'objet de l'invention, dont ils ne constituent en aucune manière une limitation.

**EXEMPLE 1 : Matériels et Méthodes.**

1-Souches bactériennes, plasmides et phages parentaux

**TABLEAU I**

| Souche | Principaux caractères | Référence |
|---|---|---|
| HB2151 | *ara Δ(lac-pro) thi / F' proA$^+$B$^+$ .lacI$^q$ZΔM15* | Carter et al., 1985 |
| RZ1032 | *Hfr dut ung r$^+$ m$^+$* | Kunkel et al., 1987 |
| BL21 (DE3) | *F$^-$ ompT hsdS$_B$(rB$^-$,m$_B$$^-$) gal dcm / lambdaDE3* | Studier et Moffatt, 1986 |

Le vecteur pASK98-D1.3 code pour un hybride entre un scFv de l'anticorps monoclonal mAbD1.3 et une étiquette de type streptavidine. Le gène correspondant est sous contrôle du promoteur et de l'opérateur tétracycline (Skerra, 1994). pSPI1.0 est un dérivé de pET26b (Novagen) et permet le sous-clonage des scFv sous contrôle du promoteur du phage T7.

**2-Milieux de culture et tampons**

Les milieux LB et 2xYT ont été décrits (Sambrook et al., 1989). Le milieu SB contient 16 g/l bacto-tryptone, 10 g/l de bacto-levure, 10 g/l NaCl, 50 mM $K_2HPO_4$, 5 mM $MgSO_4$ et 1 % glucose (Plückthun et al., 1996). L'ampicilline est utilisée à 200 μg/ml et la kanamycine à 30 μg/ml dans les milieux de croissance. Les tampons PBS et TBE ont été décrits (Sambrook et al, précité). Tampon P : 20 mM Tris-HCl pH7,9 ; 500 mM NaCl. Tampon Q : 50 mM Tris-HCl pH7,5 ; 150 mM NaCl. Tampon C : 50 mM phosphate de sodium pH7,0 ; 150 mM NaCl.

**3-Oligonucléotides**

Les séquences des oligonucléotides utilisés pour la mutagenèse ou les constructions génétiques sont données dans le tableau suivant. Les codons modifiés sont notées en gras pour les oligonucléotides mutagènes.

**TABLEAU II**

| Nom | Séquence | |
|---|---|---|
| pMR1-VL-H30C | 5'-atggtacgatttattaa**aca**ttataagggtg-3' | SEQ ID NO :20 |
| pMR1-VL-N31C | 5'-catgctaaata**aca**gtggatattcccacttg-3' | SEQ ID NO :17 |
| pMR1-VL-Y49C | 5'-agacgattccaacaacatat**aca**ctggtcctcga-3' | SEQ ID NO :21 |
| pMR1-VL-Y50C | 5'gattcca**aca**acatatctggtcctcgactcctcta-3' | SEQ ID NO :22 |
| pMR1-VL-T52C | 5'-catctgctaaggt**aca**tgtataatagac-3 | SEQ ID NO :13 |
| pMR1-VL-T53C | 5'-catcgctaa**gca**tgttgtataataga-3' | SEQ ID NO :1 |
| pMR1-VL-D56C | 5'-cttgatggcacacc**gca**tgctaaggttgttg-3' | SEQ ID NO :16 |
| pMR1-VL-W52C | 5'-gaggagtac**gca**aaaatgttgacagtaa-3' | SEQ ID NO :12 |
| pMR1-VL-S93C | 5'-cgaggag**taca**ccaaaaatgttgacagta-3' | SEQ ID NO :2 |
| pMR1-VL-T94C | 5'-cgtccgagg**aca**actccaaaaatgtt-3' | SEQ ID NO :3 |
| pMR1-VH-S28C | 5'-acaccatagccagttaa**gca**gaaccctgagacg-3' | SEQ ID NO :18 |
| pMR1-VH-G31C | 5'-caagagtaattgg**aca**ataccacatttg-3' | SEQ ID NO :24 |
| pMR1-VH-T30C | 5'-acaccatagcc**gca**taatgagaaccctg-3' | SEQ ID NO :15 |
| pMR1-VH-Y32C | 5'-cagtttacacc**aca**cccggttaatgaga-3' | SEQ ID NO :14 |
| pMR1-VH-G53C | 5'-acccttactaaac**aca**actacctttgtgtctg-3' | SEQ ID NO :25 |
| pMR1-VH-N56C | 5'-ttatagtctgtgcacccatcaccccaaatcat-3' | SEQ ID NO :19 |
| pMR1-VH-R99C | 5'-tgacacggtctctc**aca**ctaatatccgaactgatga-3' | SEQ ID NO :23 |
| Séquençage: | | |
| VL-CDR1-Rev | 5'-agaatattgtgttcctga-3' | SEQ ID NO :4 |
| VH-CDR1-Rev | 5'-tgctgatgctcagtctgg-3' | SEQ ID NO :5 |
| VH-Seq-CDR3 | 5'-ggtgatggaaacacagac-3' | SEQ ID NO :6 |

(suite)

| Nom | Séquence | |
|---|---|---|
| pASK98-seq-His | 5'-cgccgcgcttaatgc-3' | SEQ ID NO :7 |
| Construction de pMR1 : | | |
| pASK98-His6-For : | 5'-tcgagatcaagcggccgctggaacaccatcaccatcaccatta-3'SEQ | ID NO :8 |
| pASK98-His6-Back : | 5'-agcttaatggtgatggtgatggtggtccagcggccgcttgatc-3' | SEQ ID NO :9 |

## 4-Techniques d'ADN recombinant

*Préparation d'ADN plasmidique :* les mini-préparations d'ADN plasmidiques sont effectuées à partir de 5 ml de culture bactérienne par la méthode de la lyse alcaline (Sambrook et al., précité). Les midi-préparations sont effectuées à partir de 30 à 60 ml de culture au moyen du kit QIAFilter Plasmid Midikit (QIAgen).

*Transformation :* la préparation de bactéries compétentes par la méthode simple au $CaCl_2$ et la transformation par choc thermique sont effectuées comme décrit dans Sambrook et al., précité.

*Purification de fragments d'ADN par électrophorèse :* le mélange de restriction est chargé sur un gel d'agarose à 1 à 2 % (Easy Bag Agarose, Quantum Bioprobe). L'électrophorèse est menée 1 à 2 heures à 8 V/cm dans du tampon TBE. Le gel est ensuite coloré au bromure d'éthidium à 1 μg/ml dans l'eau. La bande d'agarose contenant le fragment d'ADN est découpée sous lampe U.V puis l'ADN est extrait et purifié au moyen du kit QIAquick Gel Extraction Kit (QIAgen).

*Ligation d'un fragment d'ADN dans un vecteur plasmidique :* pour la ligation de fragments de restriction, leur mélange est précipité avec Precipitator (Appligene), resuspendu dans l'eau puis porté 5 minutes à 50°C. 400UI de ligase à ADN de T4 (New England Biolabs) et son tampon sont ajoutés et la ligation est poursuivie toute la nuit à 16°C. Les souches HB2151 sont ensuite transformées avec les produits de ligation.

*Mutagenèse :* la mutagenèse dirigée est effectuée comme décrit dans Kunkel et al., (1987) en utilisant la polynucléotide kinase de T4, la ligase à ADN de T4 et la polymérase à ADN de T4 (New England Biolabs).

*Séquençage :* la présence de mutation ou le contrôle des constructions génétiques sont effectués par séquençage au moyen du T7-Sequencing Kit (Pharmacia), avec les mélanges de nucléotides pour lecture courte, du dATP-$^{35}$S et une amorce. La matrice est constituée par une mini-préparation d'ADN plasmidique, dénaturée par la soude puis neutralisée par passage sur colonne Microspin S-400-HR. Les gels de séquences (6 % acrylamide 1:19 bis, 42 % urée en TBE, 30 x 40 cm) sont soumis à une pré-électrophorèse de 45 min à 40 W, puis chargés avec les échantillons préalablement bouillis (Sambrook et al, précité). La migration est conduite à 40 W pendant 2 à 3 h. Les gels sont séchés et exposés une nuit avec un film BioMax MR (Kodak).

## 5-Constructions des plasmides recombinants

*pMR1 :* le vecteur pASK98-D1.3 est coupé par les enzymes XhoI et HindIII. Le mélange de restriction est passé sur colonne Microspin S-400-HR pour retirer le plus petit fragment de restriction (43 pb). Les oligonucléotides pASK98-his6-for et pASK98-his6-back sont phosphorylés et hybridés à 60°C pour former un adaptateur à extrémités cohésives qui est inséré dans le grand fragment XhoI-HindIII de pASK98-D1.3. Le phagemide obtenu, pMR1 est contrôlé par analyse de restriction avec les enzymes NotI et PvuI et par séquençage au moyen de l'oligonucléotide pASK98-seq-His6, il correspond à la SEQ ID NO :10.

*PMR 5 :* les plasmides pFBX et pSPI1.0 sont coupés par SfiI et NotI. Le petit fragment de pFBX (750 pb) contenant le gène du scFv de D1.3 et le grand fragment de pSPI1.0 (4650 pb) sont purifiés sur gel d'agarose puis assemblés par ligation. Le plasmide obtenu, pMR5 est contrôlé par analyse de restriction avec l'enzyme AFIIII.

## 6-Production des fragments d'anticorps

*Production en faible quantité à partir de pMR1 ou pMR5 :* 200 ml de milieu 2xYT ampicilline sont inoculés avec une colonie isolée des souches recom-binantes HB2151(pMR1) ou BL21(DE3, pMR5). La culture est agitée à 22, 30 ou 37°C jusqu'à l'obtention de $A_{600nm}$ = 0,5 où elle est induite avec 0,2 à 1,0 μg/ml d'anhydrotétracycline pour HB2151 ou avec 0,2 à 1,0 mM d'IPTG pour BL21(DE3) puis l'agitation est poursuivie pendant 2 h, 5 h ou 16 h. Un extrait périplasmique est préparé par choc osmotique (voir ci-dessous) à partir de 50 ml de culture centrifugés 20 min à 10500 g, à 4°C.

*Production de quantités importantes :* pour produire les scFv-His6, du milieu SB (100 ml) additionné d'ampicilline est inoculé avec une colonie isolée de la souche recombinante HB2151(pMR1). La culture est agitée pendant une nuit à 37°C puis 25 ml du contenu sont transférés dans 750 ml du même milieu pré-équilibré à 22°C. La culture est agitée à 22°C jusqu'à $A_{600nm}$ =2,0 puis induite par 0,22 μg/ml anhydrotétracycline. La croissance est poursuivie pendant environ 2 h et 30 min, jusqu'à $A_{600nm}$ = 4. La culture est centrifugée 20 minutes à 9500 g, à 4°C. L'extrait périplasmique est préparé par la méthode à la polymyxine décrite ci-après (exemple 1-7).

**7-Préparation d'extraits périplasmiques**

Les cultures bactériennes sont centrifugées et des extraits périplasmiques sont préparés à partir des culots bactériens par l'une des quatre méthodes suivantes. Les volumes sont indiqués par rapport au volume de culture traité.

*Méthode du choc osmotique :* le culot bactérien est resuspendu dans 20 % Saccharose dans 100 mM Tris-HCl pH 7,5 (1/20e volume). La suspension est maintenue 10 minutes sur la glace puis centrifugée 15 minutes à 10.000 g, à 4°C. Le culot est repris dans 0,5 mM $MgCl_2$ (1/20e volume). La suspension est de nouveau maintenue 10 minutes sur glace et centrifugée de même. Ce second surnageant constitue l'extrait périplasmique proprement dit. Le premier surnageant peut néanmoins être utilisé après dialyse contre du tampon P.

*Méthode au Tris-EDTA :* le culot bactérien est resuspendu dans 100 mM Tris-HCl pH7,5 ; 1 mM EDTA (1/20e volume). La suspension est homogénéisée 30 minutes par agitation magnétique à 4°C, puis centrifugée 30 minutes à 35000 g. Le surnageant constitue l'extrait périplasmique. Le culot est resuspendu dans 1/20e volume de SDS à 1% dans l'eau. Il contient les protéines insolubles, membranaires ou cytoplasmiques.

*Méthode Tris-EDTA-NaCl :* cette méthode est identique à la méthode au Tris-EDTA sauf que la resuspension du culot bactérien se fait dans 100 mM Tris-HCl pH7,5 ; 1 mM EDTA et 1M NaCl.

*Méthode à la polymyxine :* cette méthode est identique à la méthode au Tris-EDTA-NaCl à la seule différence que la resuspension du culot bactérien se fait dans du tampon P contenant 1 mg/ml de sulfate de polymyxine B (ICN).

**8-Purification de fragments d'anticorps**

*Purification des scFv-His6 :* l'extrait périplasmique est filtré sur filtre Millex (0,45 μm de porosité, Millipore) puis chargé sur une colonne contenant 1,5 ml de résine Ni-NTA Superflow (QIAgen) équilibrée avec 10 ml de Tampon P. La colonne est successivement lavée avec 10 ml de 5 mM imidazole, 20 mM imidazole, 40 mM imidazole en tampon P. Les scFv-His6 sont élués par 4 x 1,5 ml de 100 mM imidazole en tampon P. Les fractions d'élution sont analysées par SDS-PAGE à 15 %, selon le protocole décrit à l'exemple 1-10. Leur concentration en protéine est mesurée soit au moyen d'un réactif de Bradford (BioRad) en utilisant la serum albumine bovine (BSA) comme étalon, soit au moyen de $A_{280nm}$ en prenant $\varepsilon$ = 51 130 $M^{-1}.cm^{-1}$, calculé comme décrit dans Pace et al. (1995).

**9-Quantification de scFv-His6 par ELISA indirect**

Une plaque de microtitration (Nunc) est recouverte de lysozyme de blanc d'oeuf de poule (HEL, 10 μg/ml) en tampon 50 mM $NaHCO_3$ pH9,6 et incubée une nuit à température ambiante. La plaque est saturée pendant 2 h à 37°C par de la BSA (3 % poids/volume) en tampon PBS. Les échantillons sont dilués au moins 2 fois dans du PBS à 3 % BSA, déposés sur la plaque et laissés 1 h à température ambiante. La plaque est lavée 5 fois avec du PBS à 0,05 % Tween 20, puis incubée pendant 1 h en présence d'un anticorps primaire (anticorps monoclonal de souris anti-His5, QIAgen) dilué à 0,2 μg/ml dans du PBS à 3 % BSA. La plaque est lavée comme précédemment, puis incubée pendant 1 h, en présence d'un anticorps secondaire (anticorps monoclonal anti-Fc de souris, couplé à la phosphatase alcaline) dilué au 1/10.000. La plaque est lavée comme précédemment et les complexes immuns sont révélés avec 2 mg/ml de p-nitrophénylphosphate (Sigma) dans 1M diéthanolamine-HCl pH9,8, 10 mM $MgSO_4$. Le signal $A_{405nm}$ est mesuré sur un lecteur de microplaques Labsystem Multiskan MS. Dans les conditions expérimentales utilisées, la zone de linéarité est obtenue pour des concentrations de scFv-His6 inférieures à 0,8 μg/ml.

**10-Electrophorèse des protéines et techniques de Western-blot ou d'immuno-empreinte.**

*SDS-PAGE :* Le gel de concentration est un gel 4 % acrylamide:bisacrylamide (1:29) dans 125 mM Tris-HCl pH6,8 ; 0,1 % SDS et le gel de séparation est un gel 15 % acrylamide dans 375 mM Tris-HCl pH8,8 ; 0,1% SDS. Le tampon d'électrophorèse contient 190 mM Glycine, 25 mM Tris-base et 0,1 % SDS. Les gels (8 x 7 cm) sont coulés et l'électrophorèse est réalisée à l'aide d'un appareil Hoefer Mighty Small II. Les échantillons sont additionnés de tampon de charge 2x (125 mM Tris-HCl pH8,0 ; 2,5 % SDS ; 20 % Glycérol ; 2 mg/ml bleu de bromophénol, et 10 % 2-mercaptoéthanol dans le cas des gels SDS réducteurs), et chauffés 5 min à 90°C avant d'être chargés. L'électrophorèse est réalisée pendant 1h30 à 30 mA. Les marqueurs ont les masses moléculaires suivantes: 21,5 ; 31 ; 45 ; 66,2, et 97,4 kDa (Pre-stained Molecular Weight Marker low range, Bio-Rad). Les protéines sont révélées par coloration du gel dans un bain de 1 mg/ml bleu de Coomassie R250, 42 % méthanol, 16 % acide acétique puis décoloration dans plusieurs bains de 40 % méthanol, 10 % acide acétique.

*Technique de Western-blot :* Les protéines sont séparées par SDS-PAGE puis transférées sur une membrane de nitrocellulose Hybond-C (Amersham), pendant une nuit, sous 10V, dans une cuve TE22 Series Transphor Electrophoresis Unit HSI (Hoefer), contenant 25 mM Tris-base, 190 mM glycine, 20% méthanol. Ensuite, la membrane est saturée par 3 % BSA (poids:volume) en tampon TBS (100 mM Tris-HCl pH7,5 ; 150 mM NaCl), puis incubée pendant 1 h à température ambiante avec les anticorps primaires et secondaires, dilués dans du TBS à 3 % BSA, aux concentrations décrites pour l'ELISA (exemple 1-9). Après chaque incubation avec un anticorps, la membrane est lavée dans deux bains de tampon TTT (200 mM Tris-HCl pH7,5 ; 500 mM NaCl; 0,05 % Tween 20 ; 0,2 % Triton X-100) suivi d'un bain de tampon TBS. La révélation des protéines se fait en incubant la membrane dans 100 mM Tris-HCl pH9,5 ; 100 mM NaCl ; 50 mM $MgCl_2$ ; 0,4 mg/ml Nitro Blue Tetrazolium (Sigma) et 0,2 mg/ml 5-bromo-4-chloro-3-indolylphosphate (Sigma). Elle est arrêtée en plongeant la membrane dans l'eau.

**11-Analyse de l'interaction entre scFv de mAbD1.3 et le lysozyme de poule au moyen du BIAcore**

Les mesures sont faites sur un puce de CM5 (BIACORE AB) à surface de carboxyméthyldextran, comme décrit dans England et al., (1999). Brièvement, le lysozyme est immobilisé dans un canal par liaison amide jusqu'à un niveau de 500 RU (1 RU = 1 ng/$mm^2$). Les constantes de vitesse d'association ($k_{on}$) et de dissociation ($k_{off}$) à l'interface, ainsi que leur rapport $K'_D$ (constante de dissociation à l'interface), sont mesurées à 20°C avec un flux de 10 µl/min de scFv-His6 en tampon PBS additionné de 0,005 % Tween 20. Le tampon contient en plus 1 µM de lysozyme durant la phase de dissociation pour éviter la refixation des scFv-His6 au lysozyme immobilisé. Un canal sans lysozyme immobilisé donne le signal de fixation non spécifique.

**12-Chromatosraphie d'exclusion**

Le scFv-His6 est dialysé contre du tampon Q puis concentré par ultrafiltration sur Centricon$^R$ 10 (Amicon). Les chromatographies sont réalisées au moyen d'une colonne Superdex$^R$ 75 HR10/30 (Pharmacia), en tampon Q, à un flux de 0,5 ml/min. La colonne est pré-équilibrée dans les mêmes conditions. Les protéines sont détectées dans l'effluent de la colonne au moyen de $A_{280nm}$. Les échantillons préparés en tampon Q sont injectés au moyen d'une boucle de 200 µl. Ils ont les compositions suivantes : scFv-His6, 200 µL à 50 µg/ml ; BSA, 40 µg ; chymotrypsi-nogène, 14 µg ; Acétone, 0,1 %. Le rapport des concentrations entre dimère et monomère est déterminé d'après la surface des pics obtenus.

**13-Couplage de fluorophores sur la cystéine libre d'un scFv-His6 mutant**

Les scFv-His6 mutants fraîchement purifiés et conservés rigoureusement à 4°C sont dialysés pendant une nuit contre du tampon C. Alternativement, les scFv-His6 mutants purifiés sont ajustés à une concentration d'au moins 100 µg/ml par ultrafiltration sur filtre Centricon$^R$ 10 (Amicon) puis réduits par addition de 2-mercaptoéthanol à une concentration finale de 0,1 mM, 1 mM, 10 mM ou 50 mM. Le mélange est incubé pendant 1 heure à 22°C. La protéine réduite est séparée du réducteur en excès par désalage, au moyen d'une colonne HiTrap Desalting (Pharmacia) prééquilibrée en tampon C fortement dégazé.

Le couplage est réalisé par addition de chlorure de NBD, d'ester d'IANBD, de 5-IAF ou d'acrylodan (BioProbe) dans un rapport molaire (protéine:fluorophore) d'au moins 1:20. Le mélange est incubé 24 heures à 22°C puis centrifugé au moins 15 minutes à 10.000 g, à 4°C. Le scFv-His6 marqué est ensuite repurifié. Pour cela, le mélange réactionnel est dilué 7 fois dans 5 mM imidazole en tampon P et chargé successivement 3 fois sur une colonne contenant 500 µl de résine Ni-NTA. Le fluorophore en excès est éliminé en lavant 6 fois par 5 ml de 5 mM imidazole en tampon P. Le scFv-His6 marqué est élué par 1 ml de 40 mM imidazole en tampon P puis soit par 4 fois 500 µl de 100 mM imidazole en tampon P, soit par 3 fois 600 µl de 160 mM imidazole en tampon P. Un échantillon de référence est obtenu en traitant du tampon C seul d'une manière identique. Il est utilisé comme contrôle pour les dosages protéiques et les mesures de fluorescence. Le rendement de couplage peut être estimé par le rapport des absorbances du fluorophore et de la protéine :

$\varepsilon_{280nm}$(scFv-His6) = 51,13 $mM^{-1}.cm^{-1}$, $\varepsilon_{435nm}$ (CNBD) = 9,6 $mM^{-1}.cm^{-1}$, $\varepsilon_{500nm}$ (IANBD) = 31,1 $mM^{-1}.cm^{-1}$, $\varepsilon_{492nm}$ (5-IAF) = 75 $mM^{-1}.cm^{-1}$, $\varepsilon_{391nm}$ (acrylodan) = 20 $mM^{-1}.cm^{-1}$, comme décrit dans Haugland et al., (1996).

**EXEMPLE 2: Exemple de mise en oeuvre du procédé selon l'invention.**

**1-Recherche des résidus du fragment Fv libre de mAbD1.3 utilisables pour le couplage du fluorophore (figure 1).**

La structure du fragment Fv libre de mAbD1.3 et celle de son complexe avec le lysozyme sont connues (Bhat et al., 1994). Néanmoins, pour simplifier la recherche des résidus du Fv qui sont situés au voisinage du site actif de liaison au lysozyme et utilisables pour le couplage du fluorophore, et pour rendre cette recherche applicable à d'autres couples de macromolécules pour lesquels seule la structure du complexe existe, les données expérimentales concernant le Fv libre de mAbD1.3 n'ont pas été utilisées. Un modèle structural du Fv libre a été construit en délétant les coordonnées cristallographiques du lysozyme dans la structure du complexe. Les structures ont été analysées avec la suite de logiciels WHAT IF (Vriends, 1990 ; http://www.sander.embl-heidelberg.de/whatif/). Les principes suivants ont été utilisés :

1- Trois types de résidus ont été recherchés dans le mAbD1.3 : ceux qui sont en contact direct avec le lysozyme, ceux qui sont en contact par l'intermédiaire d'une molécule d'eau, et ceux dont la surface accessible au solvant (ASA) est modifiée par la fixation du lysozyme lorsqu'on utilise des sphères de rayon 1,4 ; 1,7 ; 2,0 ; 2,3 ; 2,6 ou 2,9 Å pour la molécule de solvant. L'utilisation de rayons plus élevés que celui d'une molécule d'eau (1,4 Å), permet de définir un voisinage élargi de l'antigène à la surface de l'anticorps et de tenir compte du volume important d'un fluorophore par rapport à celui d'une molécule d'eau. Les résidus de mAbD1.3 qui satisfont à l'un de ces critères, et la nature du critère utilisé sont indiqués dans les colonnes 1 et 2 de la figure 1. Les colonnes 8 et 9 de la figure 1 donnent la nature des mutations connues dans mAbD1.3 et leurs effets, sous forme d'une variation $\Delta\Delta G$ d'énergie d'interaction avec le lysozyme ;

2- Les résidus du récepteur sélectionnés en (1) doivent être changés en Cys par mutagenèse, puis couplés au fluorophore. L'atome S$\gamma$ du résidu Cys mutant doit donc être exposé au solvant dans le Fv libre, pour pouvoir être attaqué par la molécule de fluorophore. On peut supposer, en première approximation, que le S$\gamma$ du résidu Cys mutant se superpose avec l'atome en position $\gamma$ du résidu de type sauvage. Cet atome en position $\gamma$ doit donc être exposé au solvant dans la structure du Fv libre sauvage. Lorsqu'un atome est présent en position $\delta$ dans le résidu de type sauvage, il peut masquer l'atome en position $\gamma$ vis-à-vis du solvant alors que ce masquage n'existera plus après son changement en Cys. Il a donc été considéré que l'accessibilité au solvant d'un atome en position $\delta$ pouvait être informative sur celle du S$\gamma$ du résidu Cys mutant. Les valeurs d'ASA des atomes en position $\gamma$ et éventuellement $\delta$, pour chaque résidu sélectionné au point 1), sont données dans les colonnes 3 et 4 de la figure 1 (en utilisant R = 1,4 Å) ;

3- Le fluorophore peut avantageusement être couplé au S$\gamma$ du résidu de Cys par l'intermédiaire d'une chaîne aliphatique dont la longueur varie de 1 à 6 atomes de carbone pour les fluorophores les plus courants, mais peut être plus longue. Après couplage, les groupements polaires et aromatiques du fluorophore doivent préférentiellement se trouver à la périphérie de l'interface de contact entre l'anticorps et l'antigène, et non dans cette interface où ils entreraient en conflit avec l'antigène. Pour cela, il faut que le bras aliphatique du fluorophore ait accès au solvant dans la structure du complexe entre l'anticorps marqué et l'antigène. En première approximation, cette condition est remplie si l'un des groupements qui sont situés en positions $\gamma$, $\delta$ et $\varepsilon$ dans la chaîne latérale initiale est accessible au solvant dans la structure du complexe natif. L'ASA de ces groupements de chaînes latérales est donné dans le même format qu'au point 2), dans les colonnes 5, 6 et 7 de la figure 1 ;

4- Les mutations des résidus du récepteur ne doivent pas être trop délétères pour l'interaction avec l'antigène si on veut que l'anticorps marqué conserve une affinité suffisante. Les colonnes 8 et 9 de la figure 1 donnent la nature des mutations connues dans mAbD1.3 et leurs effets, sous forme d'une variation $\Delta\Delta G$ d'énergie d'interaction avec le lysozyme (England et al., 1997 et 1999 ; Hawkins et al., 1993 ; Dall'Acqua et al. 1996 ; Dall'Acqua et Carter, 1998).

La colonne 10 de la figure 1 résume les critères utilisés. Les résidus retenus sont divisés en trois classes de priorités différentes. La première classe contient les résidus dont l'atome en position $\gamma$ est accessible au solvant dans la structure du Fv libre, et qui sont en contact avec le lysozyme soit directement, soit par l'intermédiaire d'une molécule d'eau : VL-Thr53, VL-Ser93, VL-Thr94 et VH-Thr30. La deuxième classe contient les résidus dont l'atome en position $\gamma$ est accessible et qui ne sont pas en contact direct avec le lysozyme : VL-Asn31, VL-Thr52, VL-Asp56, VH-Scr28, VH-Asn56. La troisième classe contient les résidus dont l'atome en position $\delta$ est accessible, mais pas l'atome en position $\gamma$ : VL-Asn28, VL-His30,VH-Asp58, VH-Arg99.

**2-<u>Construction de vecteurs d'expression du scFv-His6 et optimisation de la production du scFv-His6</u> (figure 2)**

**a) Construction de vecteurs d'expression du scFv-His6**

L'étude de l'effet du couplage de fluorophores à des résidus du scFv de mAbD1.3 tels que définis en 1, sur la fixation au lysozyme, a été réalisée à l'aide de scFv de mAbD 1.3 recombinants possédant une extension C-terminale de 6 résidus histidine (scFv-His6), exprimés à l'aide de vecteurs d'expression procaryotes. La forme sauvage ainsi que les formes mutées de scFv ont été exprimées et purifiées sur colonne de nickel.

Deux vecteurs qui codent pour le scFv-His6, pMR1 et pMR5, ont été construits selon le protocole décrit à l'exemple 1-5. Ils portent le gène de résistance à la kanamycine ou à l'ampicilline, l'origine de réplication du phage M13 (f1IG), et des séquences d'adressage périplasmique, dérivés des gènes pelB ou ompA, en amont du gène du scFv. Le vecteur pMR5 porte le gène du scFv-His6 sous contrôle d'un promoteur du phage T7 et de l'opérateur lactose, et le gène du répresseur lactose. L'expression se fait dans la souche BL21(DE3) qui porte le gène de l'ARN polymérase de T7 sous contrôle du promoteur/opérateur lacOlacp(UV5) sur le prophage λDE3. L'addition d'IPTG dans le milieu de culture induit l'expression des gènes de l'ARN polymérase et du scFv. Le vecteur pMR1 porte le gène du scFv-His6 sous contrôle du promoteur/opérateur tétracycline ($tet^{p/o}$), et le gène du répresseur correspondant. L'induction se fait par ajout d'un analogue non toxique de la tétracycline, l'anhydrotétracycline, efficace à très faible concentration.

**b) Optimisation de la production du scFv-His6**

La production du scHis6, estimée par la quantité de scFv-His6 présente dans un extrait périplasmique brut, est mesurée par ELISA indirect au moyen d'un anticorps anti-His5, selon les techniques décrites dans l'exemple 1-9. Cette méthode permet de tester rapidement de nombreuses combinaisons de paramètres de production, tels que la température de culture, le temps d'induction et la concentration en inducteur (figure 2).

La production de faibles quantité de fragments d'anticorps à partir de pMR1 ou de pMR5, dans les conditions décrites à l'exemple 1-6, a montré que le vecteur pMR1 donnait le meilleur rendement à 22°C, en induisant 2 heures avec 0,2 μg/ml d'anhydrotétracycline. Ces mêmes conditions ont été utilisées pour produire des scFv-His6 mutants de mAbD1.3 (figure 2).

La production de quantités importantes de fragments d'anticorps à partir de pMR1 ou de pMR5, dans les conditions décrites à l'exemple 1-6, a montré que le rendement de production était meilleur lorsque l'induction avait lieu en phase exponentielle de croissance. Or à 22°C, le milieu 2xYT ne permet qu'une courte phase exponentielle de croissance pour la souche HB2151, entre $A_{600nm}$=0,3 et $A_{600nm}$=1,1 environ De plus, il a été observé que les bactéries lysaient dans ce milieu. Ce dernier a donc été complémenté par une base faible ($K_2HPO_4$), pour éviter son acidification en cours de culture, et par des composés stabilisant la membrane bactérienne externe ($MgSO_4$, glucose, NaCl). Il a été observé qu'avec le milieu résultant (milieu SB), la phase exponentielle de croissance était fortement allongée à 22°C, jusqu'à $A_{600nm}$=3,8. Ce milieu a permis d'obtenir 4 fois plus de scFv-His6 qu'en milieu 2xYT, à partir d'un volume donné de culture.

Deux types de méthodes existent pour extraire le fluide périplasmique d'une bactérie: 1) les bactéries sont équilibrées dans un milieu à forte pression osmotique puis transférées dans un tampon hypoosmotique. Le choc osmotique fait éclater la membrane externe et le contenu du périplasme est récupéré dans le second tampon ; 2) les bactéries sont resuspendues dans un tampon lysant la membrane externe (EDTA, peptides amphiphiles comme la polymyxine B). Le contenu du périplasme est alors récupéré en une seule étape. Quatre méthodes de préparation de l'extrait périplasmique de HB2151(pMR1), décrites dans l'exemple 1-7 ont été testées, et la quantité de scFv-His6 présente dans chaque extrait a été estimée par ELISA. Le produit des mesures de $A_{405nm}$ par le volume de l'extrait a donné les valeurs relatives suivantes: 100 ± 6 pour la méthode du choc osmotique (moyenne ± erreur standard sur 3 mesures), 58 ± 9 pour la méthode au Tris-EDTA, 61 ± 8 pour la méthode au Tris-EDTA-NaCl et 106 ± 9 pour la méthode à la polymyxine B. Cette dernière méthode a donné les meilleurs résultats et a donc été utilisée pour les productions des scFv-His6 sauvage et mutants en grand volume.

**3- Purification du scFv-His6**

La queue de polyhistidine des scFv-His6 permet leur purification sur colonne de nickel immobilisé Ni-NTA, selon le protocole décrit à l'exemple 1-8. Après chargement de la colonne avec l'extrait périplasmique, les lavages et l'élution se font avec différentes concentrations d'imidazole, qui entre en compétition avec les histidines pour la coordination du nickel. La concentration d'imidazole pour le lavage, qui permet de décrocher les impuretés sans déplacer le scFv-His6 lui-même, et la concentration pour l'élution qui permet de décrocher le scFv-His6 en un minimum de fractions sans le dénaturer sont précisées à l'exemple 1-8. La pureté des fractions d'élution a été analysée par électrophorèse en gel de polyacrylamide-SDS et coloration au bleu de Coomassie, selon le protocole décrit à l'exemple 1-10. Ces fractions contiennent une protéine qui a une masse moléculaire apparente égale à la masse attendue pour les scFv-His6, et sont pures à 95%. Les pertes de matériel dues à la non-fixation sur le nickel ou au décrochement au cours des lavages ont été estimées. Le produit des mesures de $A_{405nm}$ par le volume de chaque fraction a donné les valeurs relatives suivantes : des 100% contenus dans l'extrait brut, 6,7% des scFv-His6 ne se fixent pas sur la colonne, 7,6% sont décrochés au cours des lavages et 85,6% se retrouvent dans les fractions d'élution. Dans les conditions optimales de culture, d'extraction et de purification, 650 μg de scFv-His6 sauvage purifié ont été obtenus par litre de culture.

**4-Propriétés d'oligomérisation et de fixation au lysozyme du scFv-His6 de type sauvage (figure 4)**

L'appareil BIAcore permet d'obtenir rapidement les constantes de vitesse d'association $k_{on}$ et de dissociation $k_{off}$ entre anticorps et antigène, ainsi que la constante d'équilibre $K'_D=k_{off}/k_{on}$, à l'interface entre le tampon et la surface sur laquelle a été immobilisé l'antigène. Les mesures s'effectuant à flux continu sur la surface, elles peuvent être faussées par la recapture des scFv récemment dissociés sur un autre site d'antigène immobilisé. Cette recapture a peu d'influence avec un fragment d'anticorps monovalent, et elle est limitée par l'adjonction d'antigène en solution dans le tampon de dissociation. Cependant les scFv sont susceptibles de former des dimères par association du domaine VH d'une molécule avec le domaine VL d'une autre (Arndt et al., 1998). Ces dimères possèdent deux sites de fixation à l'antigène. Ils mènent à une probabilité de recapture accrue.

Avant d'effectuer les mesures sur BIAcore, il est donc nécessaire de vérifier l'état de dimérisation du scFv-His6 de mAbD1.3. Les proportions de monomère et de dimère au pH utilisé pour le BIAcore (pH7,5) et à une concentration de 50 μg/ml ont été estimées par la hauteur des pics correspondant en chromatographie d'exclusion, selon le protocole de l'exemple 1-12, et les résultats sont présentés à la figure 4. Le rapport des surfaces des pics du dimère et du monomère donne un taux de dimérisation de 9,8%. Ce taux faible permet de négliger l'effet de la recapture sur la surface lors des mesures sur BIAcore. De plus ces mesures ont été faites à des concentrations de 0,1 à 20 μg/ml, inférieures aux 50 μg/ml utilisés pour la chromatographie d'exclusion. Les paramètres cinétiques de l'interaction entre le scFv-His6 et le lysozyme, déterminés sur BIAcore, selon le protocole décrit à l'exemple 1-11, sont les suivantes :

$$k_{on} = 1{,}15\ (\pm\ 0{,}15)\ .\ 10^{5}\ M^{-1}.s^{-1}$$

$$k_{off} = 1{,}20\ (\pm\ 0{,}22)\ .\ 10^{-3}\ s^{-1}$$

$$K'_{D} = 10{,}5\ (\pm\ 3{,}3)\ nM$$

**5-<u>Construction, production et purification des scFv-His6 mutants</u> (figure 3)**

Les mutations en cystéine des résidus de l'anticorps mAbD1.3, placés dans la première classe (VH-T30C, VL-T53C, VL-S93C et VL-T94C, voir également le paragraphe 1 de cet exemple et la colonne 10 de la figure 1) ont été introduites dans le vecteur d'expression pMR1 par la méthode de Kunkel décrite à l'exemple 1-4. Les oligonucléotides utilisés, décrits à l'exemple 1-3, ont été conçus pour introduire ou supprimer des sites de restriction. La mutation est alors détectable par analyse des fragments obtenus après coupure. Les clones mutants ont été de plus séquencés dans la région de la mutation, à l'aide des oligonucléotides décrits à l'exemple 1-3, selon la méthode décrite à l'exemple 1-4, afin de vérifier l'intégrité de la séquence. Le plasmide dérivé de pMR1 portant la mutation VL-S93C, dénommé pMR1 (VL-S93C), correspond à la SEQ ID NO :11. La capacité de plusieurs dérivés mutants de pMR1 à exprimer le scFv-His6 a été testée dans les conditions décrites pour la production du scFv-His6 sauvage à partir de pMR1 (exemple 2-2). Les résultats montrent que ces mutants sont produits efficacement en comparaison du sauvage : 100 ± 12 pour le type sauvage, 100 ± 29 pour VL-S93C, 97 ± 5 pour VL-T94C. Le mutant VL-S93C a été purifié avec des rendements de 575 μg par litre de culture, comparables à ceux obtenus avec le scFv-His6 sauvage.

La pureté des fractions d'élution a été analysée par électrophorèse en gel de polyacrylamide-SDS et coloration au bleu de Coomassie, selon le protocole décrit à l'exemple 1-10. Les résultats obtenus sont présentés à la figure 3. Ces fractions contiennent une protéine qui a une masse moléculaire apparente égale à la masse attendue pour les scFv-His6, et sont pures à 95 %.

**6-<u>Absence de dimérisation covalente du mutant VL-S93C</u> (figure 5)**

La présence d'une cystéine libre sur les scFv-His6 mutants pourrait entraîner la formation de ponts disulfures intermoléculaires parasites pendant la production, la purification ou au cours du couplage du fluorophore. Le scFv-His6 (VL-S93C), dialysé contre le tampon de couplage, a été analysé par chromatographie d'exclusion dans les conditions utilisées pour le scFv-His6 de type sauvage (exemple 1-12 et exemple 2-4). Le chromatogramme ne présente qu'un seul pic, suggérant que le scFv-His6(VL-S93C) n'existe que dans un seul état d'oligomérisation dans le tampon utilisé (figure 5). La position de ce pic correspond presque à celle du scFv-His6 monomérique de type sauvage. La faible différence entre les positions de ces deux pics pourrait être due au fait que les deux chromatogrammes ont été développés dans des tampons de nature et de pH légèrement différents.

**7-Couplage de fluorophores au mutant VL-S93C (figures 6 à 10)**

Le couplage de l'IANBD (figure 6) au scFv-His6(VL-S93C) est effectué après traitement du fragment d'anticorps par différentes concentration de 2-mercaptoéthanol et désalage, selon le protocole décrit à l'exemple 1-13. La réaction est faite à pH=7,0 pour éviter le couplage parasite sur des lysines déprotonées à pH basique (Houk et al., 1983 ; Del Boccio et al., 1991). Après couplage, le fluorophore en excès et le dérivé du scFv-His6(VL-S93C) sont séparés par une nouvelle purification sur colonne de Nickel. Plusieurs arguments convergent pour confirmer la spécificité du couplage sur la cystéine VL-Cys93 du scFv mutant. Lors de la repurification, le fluorophore est efficacement lavé et la fluorescence résiduelle est éluée de la colonne en même temps que le scFv. Dans les fractions d'élution, l'intensité de fluorescence est proportionnelle à la quantité de protéine, ce qui suggère un couplage stoechiométrique (figure 7).

En l'absence de traitement réducteur, le scFv-His6 de type sauvage ne réagit pratiquement pas avec l'IANBD, alors que le scFv-His6(VL-S93C) est marqué à 8% par le fluorophore (figure 8). Ce dernier rendement est fortement amélioré lorsque le scFv-His6(VL-S93C) est réduit par le 2-mercaptoéthanol, rapidement desalé et mis en présence du IANBD. En contrepartie, ce traitement occasionne une perte de matériel d'autant plus importante que la réduction est poussée. Il est probable que le scFv-His6 soit rendu en partie insoluble par la réduction de ses ponts disulfures. La perte de matériel lors des étapes de désalage (-30%) et de repurification (-15%) intervient également dans le rendement global du couplage. La concentration de 10 mM en 2-mercaptoéthanol a permis d'obtenir des rendements de couplages satisfaisants avec deux mutants différents (figures 9 et 10), tout en préservant suffisamment de matériel pour les mesures de fluorescence.

**8-Variations de la fluorescence du mutant VL-S93C couplé à un fluorophore en fonction de la concentration en antigène (figure 11 et figure 12)**

Un spectre d'émission du conjugué scFv-His6(VL-S93ANBD), effectué immédiatement après l'adjonction d'antigène (lysozyme) montre une exaltation de la fluorescence qui dépend de la concentration finale en antigène. L'intensité de fluorescence sature à 1,26 $\pm$ 0,05 fois celle du biocapteur libre lorsque la concentration en antigène est suffisante, et de manière reproductible sur plusieurs préparations de biocapteurs. Aucun déplacement significatif de la longueur d'onde du maximum d'émission n'est observée (figure 11).

Les intensités de fluorescence ont été enregistrées en l'absence ou en présence de concentrations variables de lysozyme (10 nM à 2 $\mu$M) dans un tampon 50 mM Tris-HCl, 150 mM NaCl, pH 7,5.

Les résultats présentés à la figure 12 montrent que dans ce tampon, l'intensité de fluorescence du conjugué scFv-His6 (VL-S93ANBD) est proportionnelle à la concentration de lysozyme jusqu'à 400 nM et augmentée de 91 % à saturation. La courbe obtenue permet de titrer directement le lysozyme entre 10 et 400 nM.

Par conséquent, les résultats obtenus à la figure 12 démontrent que le biocapteur selon l'invention permet avantageusement de titrer directement un antigène ou un haptène, par exemple dans un fluide corporel.

**9- Rendement de couplage et variation de la fluorescence en fonction de la concentration en antigène d'une série de mutants scFv-His6 (figure 13).**

Des mutants scFv-His6, sélectionnés selon le procédé décrit à l'exemple 2-1, ont été produits de façon similaire au mutant VL-S93C (exemple 2-5), selon les méthodes décrites à l'exemple 1 (1-1 à 1-12). Ainsi, des plasmides dérivés de pMR1 (SEQ ID NO:10) portant les mutations décrites dans la figure 12 ont été construits par mutagénèse dirigée à l'aide des oligonucléotides présentés dans le Tableau II. Les mutants scFv-His6 produits à partir de ces plasmides ont été couplés à l'IANBD, de façon similaire au mutant VL-S93C (exemple 2-7), en utilisant une concentration de mercaptoéthanol de 10 mM, selon le protocole décrit à l'exemple 1-13. Les résultats présentés à la figure 13 montrent que des rendements de couplage satisfaisants sont obtenus pour la quasi-totalité des positions de couplage sélectionnées et que des exaltations importantes de fluorescence sont observées pour plus de la moitié de ces positions de couplage.

**RÉFÉRENCES**

- Arndt K.M. et al., Biochemistry, 1998, 37, 12918-12926.
- Bhat T.N. et al., Proc. Natl. Acad. Sci. USA, 1994, 91, 1089-1096.
- Braden B.C. et al., Immunological Review, 1998, 163, 45-57.
- Carter P. et al., Nucleic Acids Research, 1985, 13, 4431-4443.
- Creighton T.E., Proteins: Structure and molecular properties (Second edition). W.H. Freeman and Cie, 1996, 227-229.

- Dall'Acqua W. et al., Biochemistry, 1996, 35, 9667-9676
- Dall'Acqua W. et al., Current Opinion in Structural Biology, 1998, 8, 443-450
- Del Boccio G.et al., The Journal of Biochemical Chemistry, 1991, 266, 13777-13782.
- England P. et al., Biochemistry, 1997, 36, 164-172
- England P. et al., The Journal of Immunology, 1999, 162, 2129-2136
- Gilardi G. et al., Analytical Chem., 1994, 66, 3840-3847.
- Glockshuber R. et al.,. Biochemistry, 1992, 31, 1270-1279.
- Goldbaum FA. et al., J. Mol. Recogn., 1996,. 9, 6-12.
- Haugland R.P, Handbook of fluorescent probes and research chemicals (sixth edition). Molecular Probes Inc., Eugene OR, 1996.
- Hawkins RE. et al., J. Mol. Biol., 1993, 234, 958-964.
- Houk W.T. et al., The Journal of Biological Chemistry, 1983, 258, 9:5419-5423.
- Ito W. et al., J. Biol. Chem., 1993, 268, 16639-16647.
- Ito W. et al., J. Mol. Biol., 1995, 248, 729-732.
- Kunkel T.A et al., Methods in Enzymology, 1987, 154, 367-382.
- Langedijk A.C. et al., Journal of Molecular Biology, 1998, 283, 95-110.
- Marvin J.S. et al., Proc. Natl. Acad. Sci. USA., 1997, 94, 4366-4371.
- Marvin J.S. et al., J. Am. Chem. Soc., 1998, 120, 7-11.
- Narazaki et al., Biochimica et Biophysica Acta, 1997, 1338, 275-281.
- Pace C.N. et al., Protein Structure, 1995, 4, 2411-2423.
- Piervincenzi RT. et al., Biosensors & Bioelectronics, 1998, 13, 305-312.
- Pollack S.J. et al., Science, 1988, 242, 1038-1040.
- Plückthun A. et al., Antibody engineering, a practical approach. McCaffert, J., Hoogenboom, H.R. and Chiswell, D.J. Eds. Oxford University Press, 1996, 203-252.
- Rees A.R. et al., Protein structure prediction, a practical approach. Sternberg M.J. E. Ed. Oxford University Press, 1996, 141-172.
- Saleemuddin M., Adv. Biochem. Eng. Biotech., 1999, 64, 203-226.
- Sambrook J. et al., Molecular cloning: a laboratory manual. Cold Spring Harbor Laboratory Press, 1989.
- Sara M. et al., Micron., 1996, 27, 141-156.
- Skerra, A., Gene, 1994, 51, 131-135.
- Sloan D.J. et al., Protein Engineering, 1998, 11,819-23.
- Sternberg M.J.E., Protein Structure Prediction, a practical approach. Oxford University Press, 1996.
- Studier F.W. et Moffatt, B.A., Journal of Molecular Biology, 1986, 189, 113-130.
- Tolosa L. et al., Analytical Biochem., 1999, 267, 114-120.
- Turkova J., J. Chromatography, 1999, 722, 11-31.
- Vriends G., Journal of Molecular Graphism, 1990, 8, 52-56.
- Weetall H.H., Appl. Biochem. Biotech., 1993, 41, 157-188.
- Yoshioka M. et al., J. Chromatography, 1991, 566, 361-368.
- Ysern X.et al., J. Mol. Biol., 1994, 238, 496-500.


## Revendications

1. Biocapteur, **caractérisé en ce qu'**il est constitué par au moins un fragment d'un récepteur de nature protéique possédant un ou plusieurs ponts disulfures indispensables à son activité ou au maintien de sa structure et apte à se lier à un ligand convenable par l'intermédiaire d'un site de liaison, lequel fragment comprenant un fluorophore couplé à l'atome en position $\gamma$ d'au moins l'un de ses résidus d'acide aminé naturellement présent sous forme de résidu Cys ou substitué en résidu Cys, lequel résidu d'acide aminé situé au voisinage des résidus du site de liaison le long de la séquence du récepteur, est sélectionné dans le groupe constitué par les résidus qui sont en contact direct avec le ligand, ceux qui sont en contact par l'intermédiaire d'une molécule d'eau, et ceux dont la surface accessible au solvant est modifiée par la fixation du ligand, et lequel biocapteur est susceptible d'être obtenu par un procédé comprenant les étapes suivantes :

(a) identification des résidus d'acide aminé du site de liaison du récepteur par mutagénèse de l'ensemble ou d'un sous-ensemble des résidus d'acide aminé du récepteur, et détermination des variations des paramètres d'interaction avec le ligand ($K_D$, $k_{on}$, $k_{off}$) qui sont dues à chaque mutation ou à des groupes limités de mutations ;
(b) sélection des résidus Cys ou des résidus à muter en cystéine parmi les résidus d'acide aminé du récepteur qui sont situés au voisinage des résidus du site de liaison le long de la séquence du récepteur ;
(c) mutation dirigée d'au moins l'un des résidus d'acide aminé sélectionné en (b), en un résidu Cys, dans le cas où ledit résidu n'est pas naturellement un résidu Cys ;
(d) réduction ménagée du récepteur obtenu en (b) ou en (c) ; et
(e) couplage de l'atome Sγ d'au moins un résidu Cys obtenu en (b) ou en (c) avec un fluorophore.

2. Biocapteur selon la revendication 1, **caractérisé en ce que** le fluorophore est sélectionné dans le groupe constitué par le *N*-((2-iodoacetoxy)ethyl)-*N*-methyl)amino-7-nitrobenz-2-oxa-1,3diazole, le 4-chloro-7-nitrobenz-2-oxa-1,3-diazole, l'acrydolan, la 5-iodoacétamidofluorescéine, ou un fluorophore possédant une chaîne aliphatique de 1 à 6 carbones.

3. Biocapteur selon la revendication 1 ou la revendication 2, **caractérisé en ce que** ledit biocapteur est sous forme soluble.

4. Biocapteur selon l'une quelconque des revendications 1 à 3, **caractérisé en ce que** ledit biocapteur est immobilisé sur un support solide convenable.

5. Biocapteur selon la revendication 4, **caractérisé en ce que** ledit support solide est sélectionné dans le groupe constitué par les microplaques et les fibres optiques.

6. Puce à base de protéines, **caractérisée en ce qu'**elle est constituée par un support solide sur lequel est immobilisé au moins un biocapteur selon l'une quelconque des revendications 1 à 5.

7. Utilisation des biocapteurs selon l'une quelconque des revendications 1 à 5, pour la détection, le dosage ou la localisation de ligands.

8. Utilisation des biocapteurs selon l'une quelconque des revendications 1 à 5, pour le criblage de banques de protéines.

9. Utilisation des biocapteurs selon l'une quelconque des revendications 1 à 5, pour le tri de molécules.

10. Utilisation des biocapteurs selon l'une quelconque des revendications 1 à 5, pour le tri de cellules.

11. Utilisation des biocapteurs selon l'une quelconque des revendications 1 à 5, pour la fabrication de puces à base de protéines.

12. Réactif de détection, de dosage ou de localisation de ligands, **caractérisé en ce qu'**il inclut au moins un biocapteur selon l'une quelconque des revendications 1 à 5.

## Claims

1. A biosensor, **characterized in that** it consists of at least a protein-type receptor fragment having one or more disulfide bridges essential to its activity or to the maintaining of its structure and capable of binding to an appropriate ligand via a binding site, which fragment comprises a fluorophore coupled with Sγ atom of at least one of its amino acid residues naturally present in the form of a Cys residue or substituted for a Cys residue, which amino acid residue located in the proximity of binding site residues along the receptor sequence, is selected from the group consisting of the residues which are in direct contact with the ligand, those which are in contact via a water molecule, and those whose solvent accessible surface is modified by ligand binding, which biosensor is obtainable by a process comprising the following steps:

(a) identification of receptor binding site amino acid residues by mutagenesis of all or a sub-group of the receptor amino acid residues and determination of variations in ligand interaction parameters ($K_D$, $k_{on}$, $k_{off}$) which are due to each mutation or limited groups of mutations;
(b) selection of Cys residues or residues to be mutated into cysteine among receptor amino acid residues which are located in the proximity of binding site residues along the receptor sequence;
(c) directed mutation of at least one of the amino acid residues selected in (b) into a Cys residue, in the event that said residue is not naturally a Cys residue;
(d) controlled reduction of the receptor obtained in (b) or in (c); and
(e) coupling of the Sγ atom of at least one Cys residue obtained in (b) or in (c) with a fluorophore.

2. Biosensor according to claim 1, **characterized in that** the fluorophore is selected from the group consisting of N-((2-iodoacetoxy)ethyl)-N-methyl)amino-7-nitrobenz-2-oxa-1,3diazole, 4-chloro-7-nitrobenz-2-oxa-1,3-diazole, acrylodan, 5-iodoacetamidofluoresceine or a fluorophore with an aliphatic chain of 1 to 6 carbon atoms.

3. Biosensor according to claim 1 or claim 2, **characterized in that** said biosensor is in soluble form.

4. Biosensor according to any one of claims 1 to 3, **characterized in that** said biosensor is immobilized onto a suitable solid support.

5. Biosensor according to claim 4, **characterized in that** said solid support is selected from the group consisting of microplates and optical fibers.

6. Protein-based chip, **characterized in that** it consists of a solid support on which at least one biosensor according to any one of claims 1 to 5 is immobilised.

7. Use of biosensors according to any one of claims 1 to 5, for ligand detection, dosage, or localisation.

8. Use of biosensors according to any one of claims 1 to 5, for screening protein libraries.

9. Use of biosensors according to any one of claims 1 to 5, for molecule sorting.

10. Use of biosensors according to any one of claims 1 to 5, for cell sorting.

11. Use of biosensors according to any one of claims 1 to 5, for the manufacturing of protein-based chips.

12. Reagent for ligand detection, dosage, or localisation, **characterized in that** it includes at least one biosensor according to any one of claims 1 to 5.

## Patentansprüche

1. Biosensor, **dadurch gekennzeichnet, dass** er aus wenigstens einem Fragment eines proteinartigen Rezeptors besteht, der ein oder mehrere Disulfidbrücken hat, die für seine Aktivität oder für die Aufrechterhaltung seiner Struktur unverzichtbar sind, und mittels einer Bindungsstelle an einen entsprechenden Liganden binden kann, wobei das Fragment ein Fluorophor umfasst, das an ein Atom in γ-Position von wenigstens einem seiner Aminosäurenreste gekoppelt ist, die natürlicherweise als Cysteinrest vorliegen oder durch ein Cysteinrest substituiert sind, wobei der Aminosäurerest, der in der Umgebung von Resten der Bindungsstelle entlang der Rezeptorsequenz liegt, ausgewählt ist aus der Gruppe bestehend aus Resten, die sich im direkten Kontakt mit dem Liganden befinden, solchen, die sich mittels eines Wassermoleküls in Kontakt befinden, und solchen, bei denen die dem Lösungsmittel zugängliche Oberfläche durch die Fixierung des Liganden abgewandelt wird, und wobei der Biosensor mittels eines Verfahrens erhalten werden kann, dass die folgenden Schritte umfasst:

(a) Identifizieren von Aminosäureresten der Bindungsstelle des Rezeptors durch Mutagenese der Gesamtmenge oder der Teilmenge der Aminosäurereste des Rezeptors und Erfassen von Änderungen der Wechselwirkungsparameter ($K_D$, $k_{on}$, $k_{off}$) mit dem Liganden, die zurückzuführen sind auf jede Mutation oder auf begrenzte Gruppen von Mutationen;
(b) Auswählen von Cys-Resten oder Resten, die am Cystein blockiert sind, und denjenigen Aminosäureresten des Rezeptors, die in der Umgebung der Reste der Bindungsstelle entlang der Rezeptorsequenz liegen;

(c) Mutation, gerichtet auf wenigstens eine der in (b) ausgewählten Aminosäurereste zu einem Cys-Rest, für den Fall, dass der Rest kein natürlicher Cys-Rest ist;
(d) schonende Reduktion des in (b) oder (c) erhaltenen Rezeptors, und
(e) Kopplung des Sγ-Atoms wenigstes eines in (b) oder (c) erhaltenen Cys-Restes mit einem Fluorophor.

2. Biosensor nach Anspruch 1, **dadurch gekennzeichnet, dass** der Fluorophor ausgewählt ist aus der Gruppe bestehend aus N-((2-lodacetoxy)ethyl)-N-methyl)amino-7-nitrobenz-2-oxa-1,3-diazol, 4-Chlor-7-nitrobenz-2-oxa-1,3-diazol, Acrylodan, 5-lodacetamidfluorescein oder einem Fluorophor, das eine aliphatische Kette mit 1 bis 6 Kohlenstoffen besitzt.

3. Biosensor nach Anspruch 1 oder Anspruch 2, **dadurch gekennzeichnet, dass** der Biosensor in löslicher Form vorliegt.

4. Biosensor nach einem der Ansprüche 1 bis 3, **dadurch gekennzeichnet, dass** der Biosensor auf einem geeigneten festen Träger immobilisiert ist.

5. Biosensor nach Anspruch 4, **dadurch gekennzeichnet, dass** der feste Träger ausgewählt ist aus der Gruppe bestehend aus Mikroplatten und optischen Fasern.

6. Chip auf Proteinbasis, **dadurch gekennzeichnet, dass** er aus einem festen Träger besteht, auf dem wenigstens ein Biosensor nach einem der Ansprüche 1 bis 5 immobilisiert ist.

7. Verwendung von Biosensoren nach einem der Ansprüche 1 bis 5 zum Nachweisen, Testen und Lokalisieren von Liganden.

8. Verwendung von Biosensoren nach einem der Ansprüche 1 bis 5 zum Screenen von Proteinbanken.

9. Verwendung von Biosensoren nach einem der Ansprüche 1 bis 5 zur Sortierung von Molekülen.

10. Verwendung von Biosensoren nach einem der Ansprüche 1 bis 5 zur Sortierung von Zellen.

11. Verwendung von Biosensoren nach einem der Ansprüche 1 bis 5 zur Herstellung von Chips auf Proteinbasis.

12. Reagens zum Nachweisen, Testen und Lokalisieren von Liganden, **dadurch gekennzeichnet, dass** es wenigstens einen Biosensor nach einem der Ansprüche 1 bis 5 umfasst.

| Résidu | voisinage (Å) | ASA dans Fv libre (%) | | ASA dans Fv::HEL (%) | | | Mutation | ΔΔG kcal/mol | Choix |
|---|---|---|---|---|---|---|---|---|---|
| | | Xγ | Xδ | Xγ | Xδ | Xε | | | |
| VL-Asn28 | 2.0 | 7.4 | 2.3, 59.8 | 7.8 | 2.5, 59.9 | | | | ? |
| VL-His30 | d | 0.0 | 81.4, 0.0 | 0.0 | 17.4, 0.0 | 41.3, 36.2 | A | 0.8 | ? |
| VL-Asn31 | 2.0 | 54.8 | 39.3, 35.6 | 54.5 | 39.2, 35.6 | | | | ± |
| VL-Tyr32 | d | 0.0 | 5.4, 0.0 | 0.0 | 5.3, 0.0 | 2.9, 11.0 | A, F | 1.7, 2.0 | . |
| VL-Tyr49 | d | 0.0 | 0.0, 17.5 | 0.0 | 0.0, 16.0 | 22.0, 0.0 | A | 0.8 | . |
| VL-Tyr50 | d | 100 | 17.7, 83.0 | 53.3 | 10.2, 14.2 | 0.0, 27.7 | A, F, N | 0.5, 0.5, 2.3 | . |
| VL-Thr52 | 1.4 | 67.6, 46.2 | | 65.0, 46.2 | | | K | 0 | ± |
| VL-Thr53 | d | 50.6, 53.8 | | 12.7, 44.4 | | | A | <0.5 | + |
| VL-Asp56 | 2.6 | 100 | 99.4, 98.8 | 100 | 99.6, 98.8 | | | | ± |
| VL-Gly68 | 2.9 | | | | | | | | |
| VL-Phe91 | d (b) | 0.0 | 0.0, 0.0 | 0.0 | 0.0, 0.0 | 0.0, 0.0 | | | . |
| VL-Trp92 | d | 0.0 | 14.5, 0.0 | 0.0 | 14.6, 0.0 | 68.3, 0.0, 0.0 | A | 3.7 | . |
| VL-Ser93 | d | 89.7 | | 56.0 | | | A | 0.3 | + |
| VL-Thr94 | i, 1.7 | 54.5, 7.2 | | 54.5, 7.2 | | | | | + |
| VH-Phe27 | 2.9 | 0.0 | 0.0, 0.0 | 0.0 | 0.0, 0.0 | 0.0, 0.0 | N(+E46Q) | 1.1 | . |
| VH-Ser28 | 2.0 | 45.0 | | 44.8 | | | | | ± |
| VH-Thr30 | i, 1.4 | 62.6, 51.7 | | 62.5, 51.7 | | | A | 0.1 | + |
| VH-Gly31 | d | | | | | | | | |
| VH-Tyr32 | d | 0.0 | 0.0, 5.3 | 0.0 | 0.0, 5.3 | 18.7, 4.9 | A, F | 1.1, 0.4 | . |
| VH-Gly33 | 1.4 | | | | | | | | |
| VH-Trp52 | d | 65.6 | 6.4, 59.5 | 40.0 | 6.4, 0.0 | 8.1, 0.0, 8.6 | A | 0.9 | . |
| VH-Gly53 | d | | | | | | | | |
| VH-Asp54 | d | 24.3 | 0.0, 83.8 | 5.7 | 0.0, 36.7 | | A | 1.9 | . |
| VH-Asn56 | 2.3 | 89.7 | 46.9, 100 | 89.2 | 47.0, 100 | | A, S | 0.2, 0.2 | ± |
| VH-Asp58 | 1.7 | 14.4 | 79.3, 26.0 | 14.3 | 79.2, 26.1 | | A | 0 | ? |
| VH-Arg97 | 2.3 | 0.0 | 0.0 | 0.0 | 0.0 | 0.0 | | | . |
| VH-Arg99 | d | 23.0 | 1.7 | 23.2 | 1.8 | 44.8 | A | 0.1 | ? |
| VH-Asp100 | d | 96.9 | 54.5, 72.4 | 6.5 | 2.8 | 1.2 | A, N | 3.1, 3.7 | . |
| VH-Tyr101 | d | 0.0 | 0.0, 12.7 | 0.0 | 0.0, 0.0 | 4.2, 7.9 | A, F | >4, 2.5 | . |
| VH-Arg102 | d | 0.8 | 1.5 | 0.9 | 1.5 | 18.6 | K, M | 1.6, 3.2 | . |

Figure 1

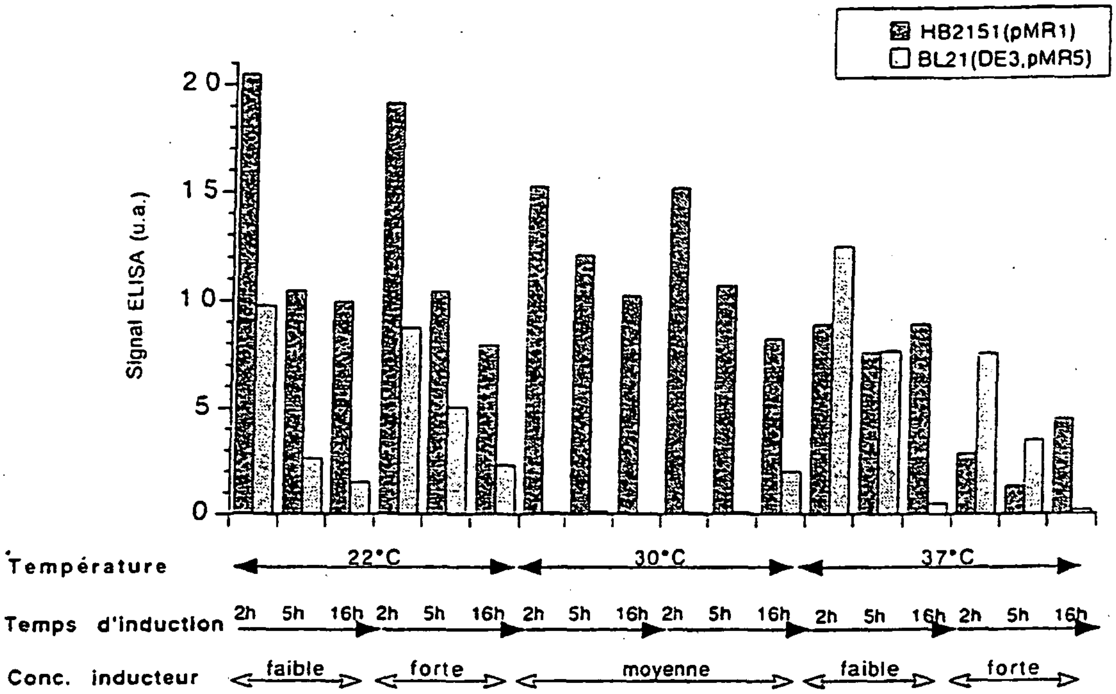
HB2151(pMR1)
BL21(DE3,pMR5)
20
15
10
5
0
Signal ELISA (u.a.)
Température
22°C
30°C
37°C
Temps d'induction
2h 5h 16h 2h 5h 16h 2h 5h 16h 2h 5h 16h 2h 5h 16h 2h 5h 16h
Conc. inducteur
faible
forte
moyenne
faible
forte

FIGURE 2

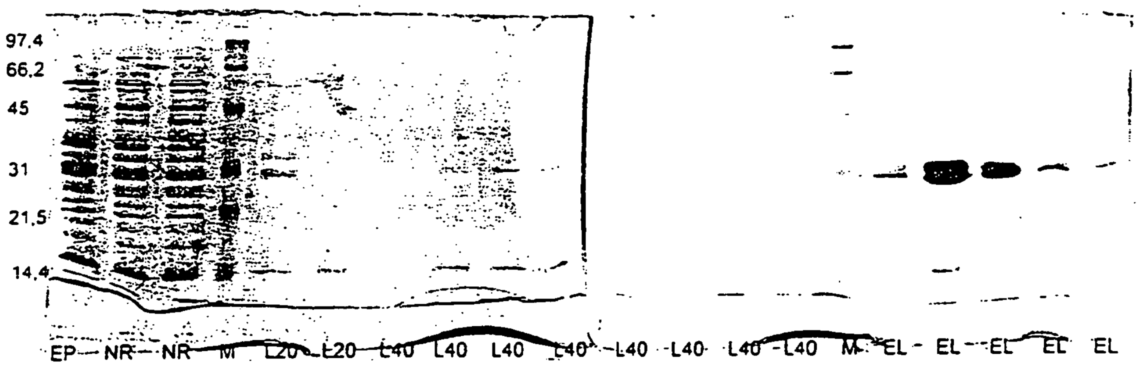
97,4
66,2
45
31
21,5
14,4
EP NR NR M L20 L20 L40 L40 L40 L40 L40 L40 L40 L40 M EL EL EL EL EL

FIGURE 3

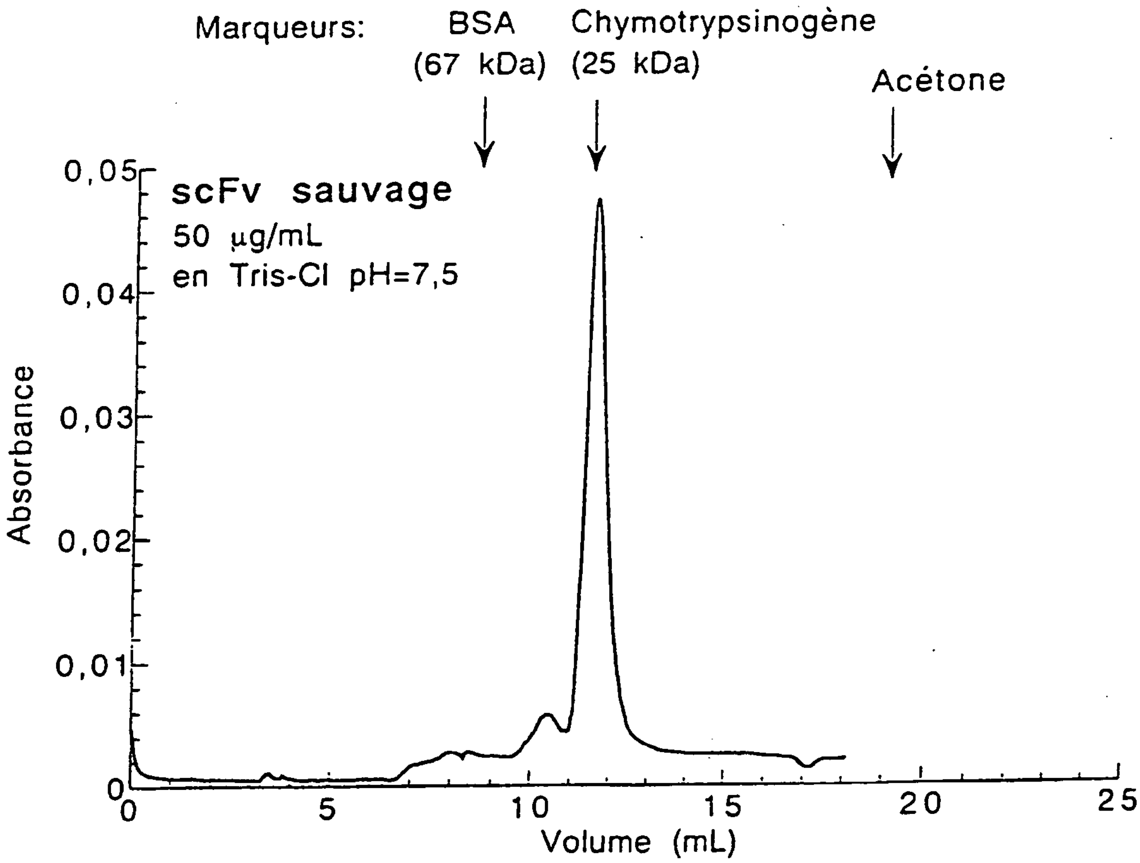
Marqueurs:
BSA
(67 kDa)
Chymotrypsinogène
(25 kDa)
Acétone
scFv sauvage
50 µg/mL
en Tris-Cl pH=7,5
Absorbance
0,05
0,04
0,03
0,02
0,01
0
0
5
10
15
20
25
Volume (mL)

FIGURE 4

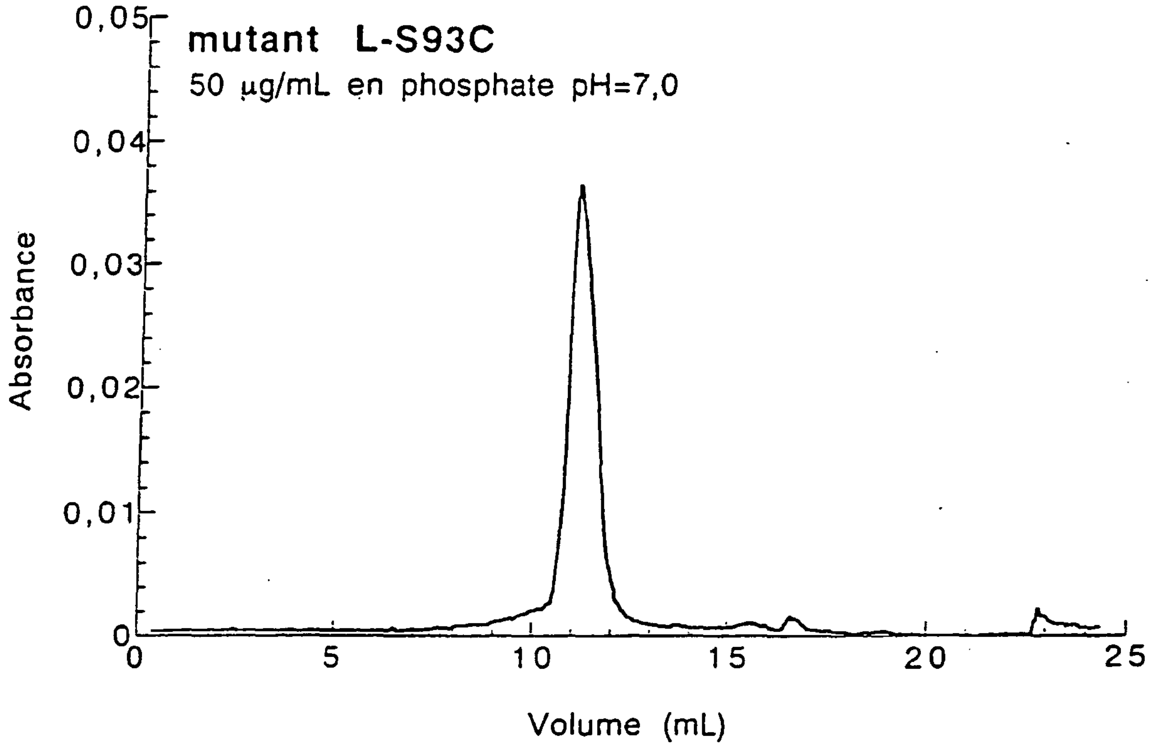
mutant L-S93C
50 µg/mL en phosphate pH=7,0
0,05
0,04
0,03
0,02
0,01
0
Absorbance
0
5
10
15
20
25
Volume (mL)

FIGURE 5

CNBD

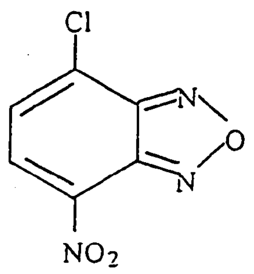

IANBD

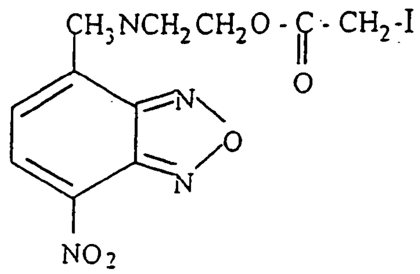

5-IAF

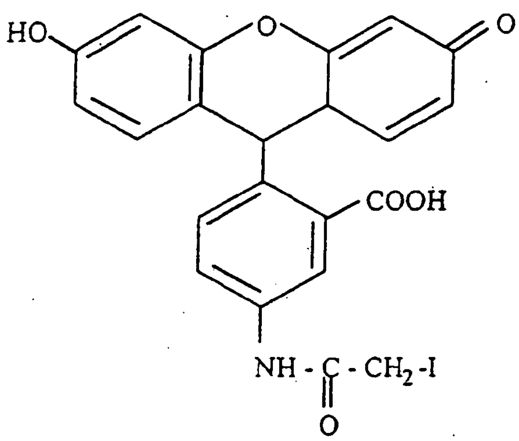

Acrylodan

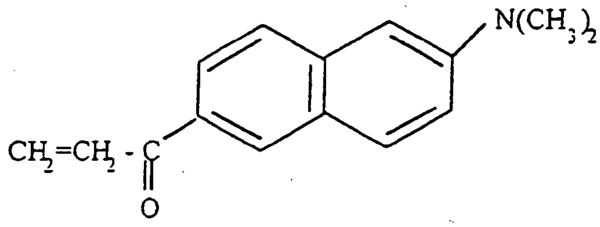

FIGURE 6

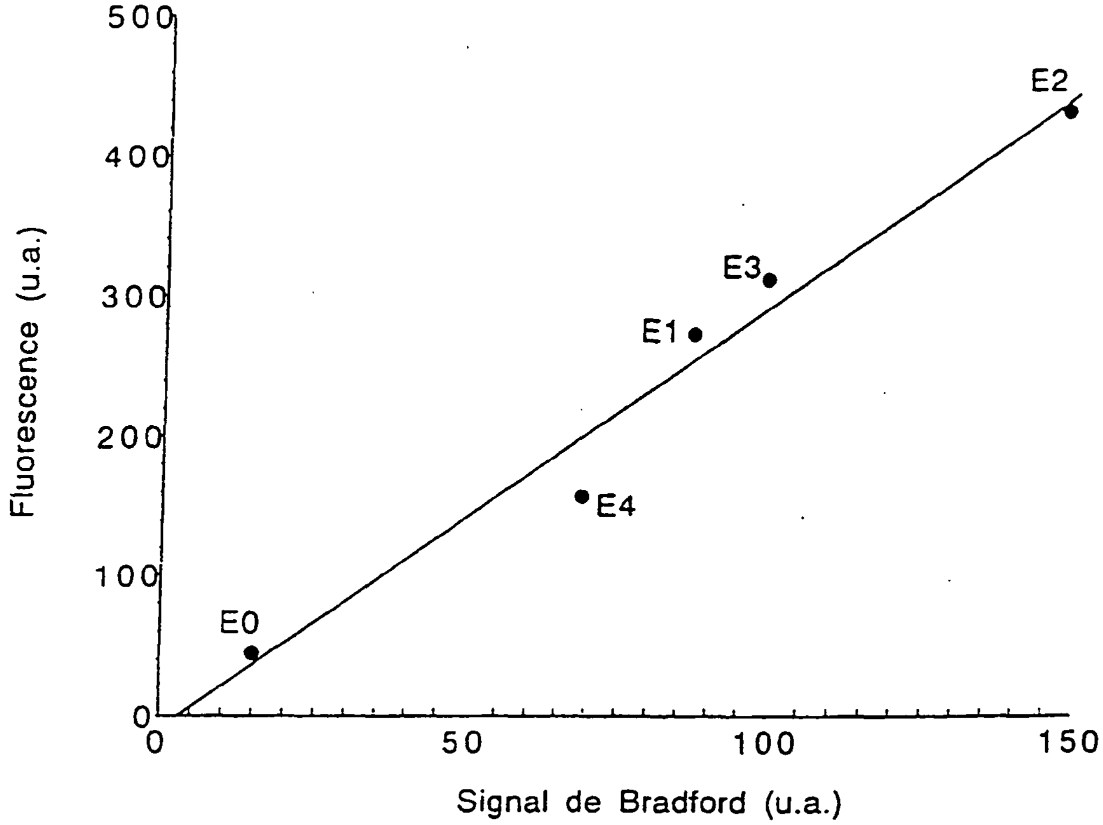
500
400
300
200
100
0
0
50
100
150
Fluorescence (u.a.)
Signal de Bradford (u.a.)
E0
E1
E2
E3
E4

FIGURE 7

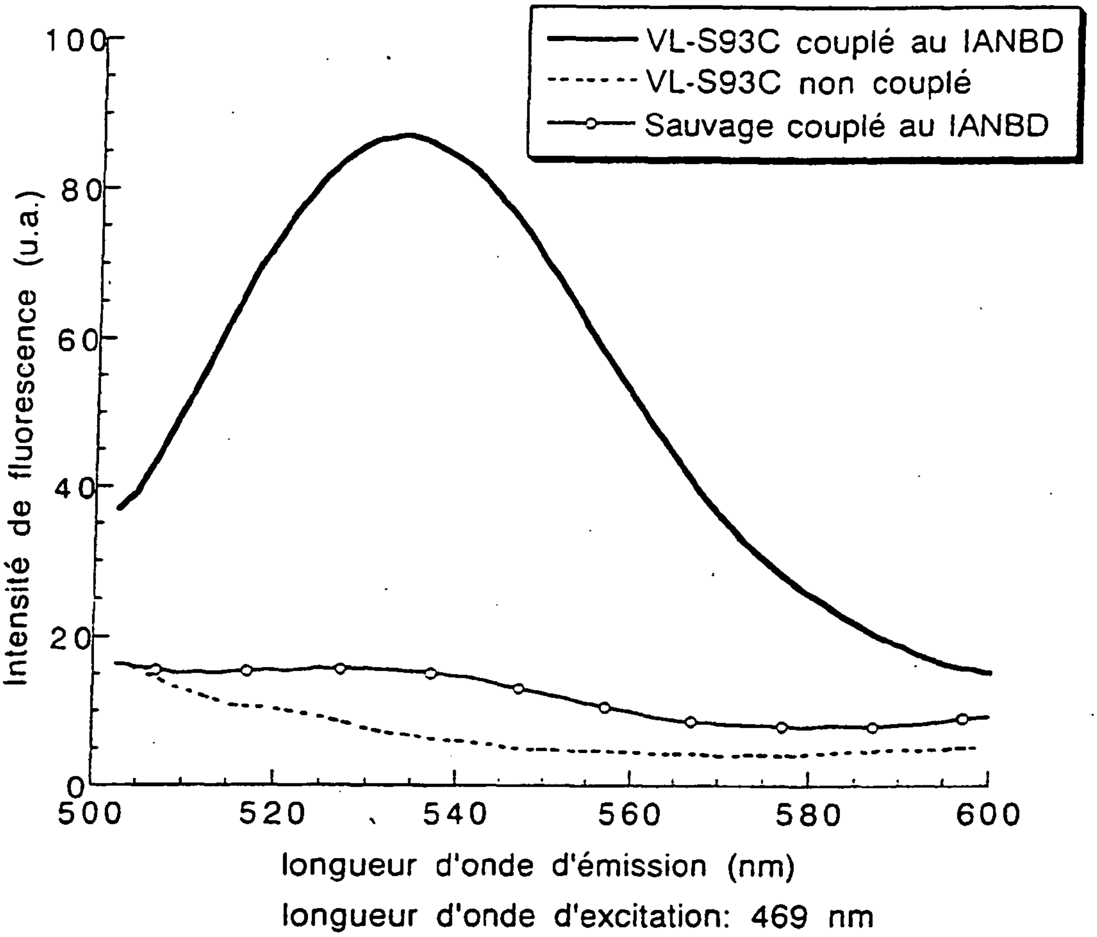
VL-S93C couplé au IANBD
VL-S93C non couplé
Sauvage couplé au IANBD
Intensité de fluorescence (u.a.)
100
80
60
40
20
0
500
520
540
560
580
600
longueur d'onde d'émission (nm)
longueur d'onde d'excitation: 469 nm

FIGURE 8

| ß-mercaptoéthanol | Mutant | Rendement de couplage | Rendement matériel |
|---|---|---|---|
| 50 mM | VH-T30C | non mesurable | 5 % |
| 10 mM | VH-T30C | 72 % | 19 % |
| 50 mM | VL-S93C | > 168 % | 4 % |
| 10 mM | VL-S93C | 83 % | 9 % |
| 1 mM | VL-S93C | 34 % | 12 % |
| 1 mM | VL-S93C | 34 % | 12 % |
| sans réduction | VL-S93C | 8 % | 85 % |

FIGURE 9

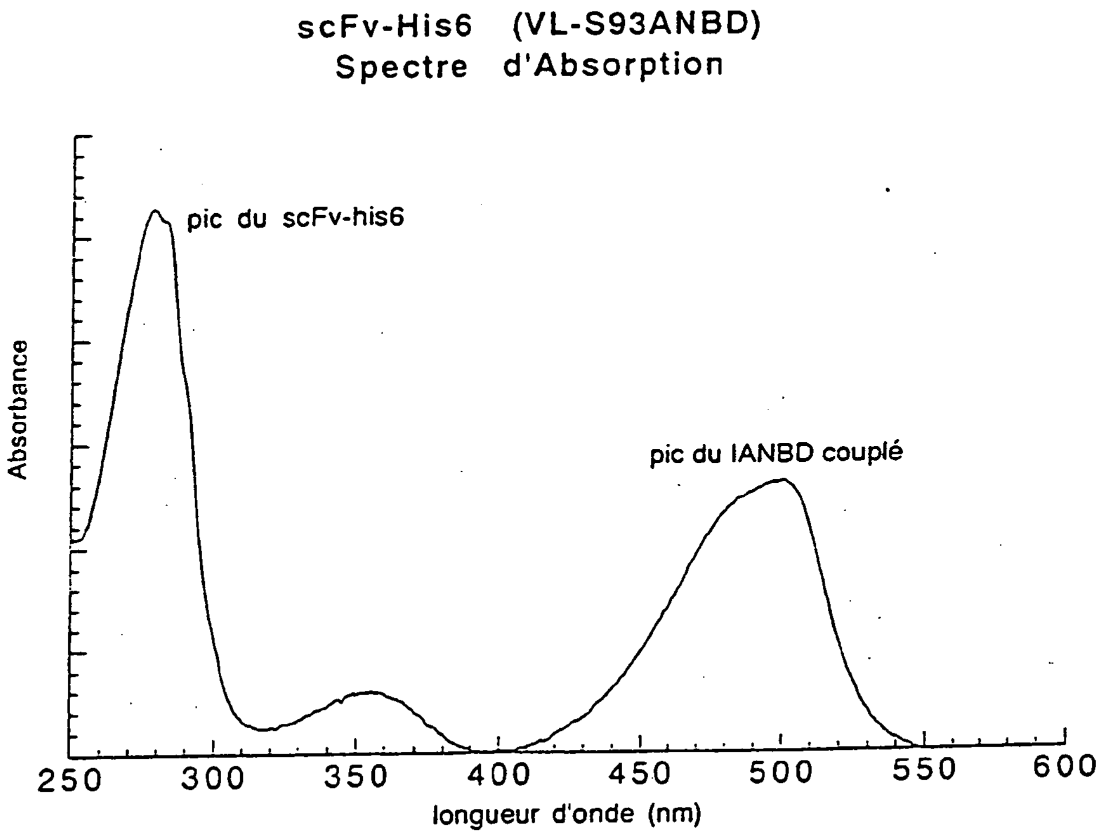
scFv-His6 (VL-S93ANBD)
Spectre d'Absorption
pic du scFv-his6
pic du IANBD couplé
Absorbance
250
300
350
400
450
500
550
600
longueur d'onde (nm)

FIGURE 10

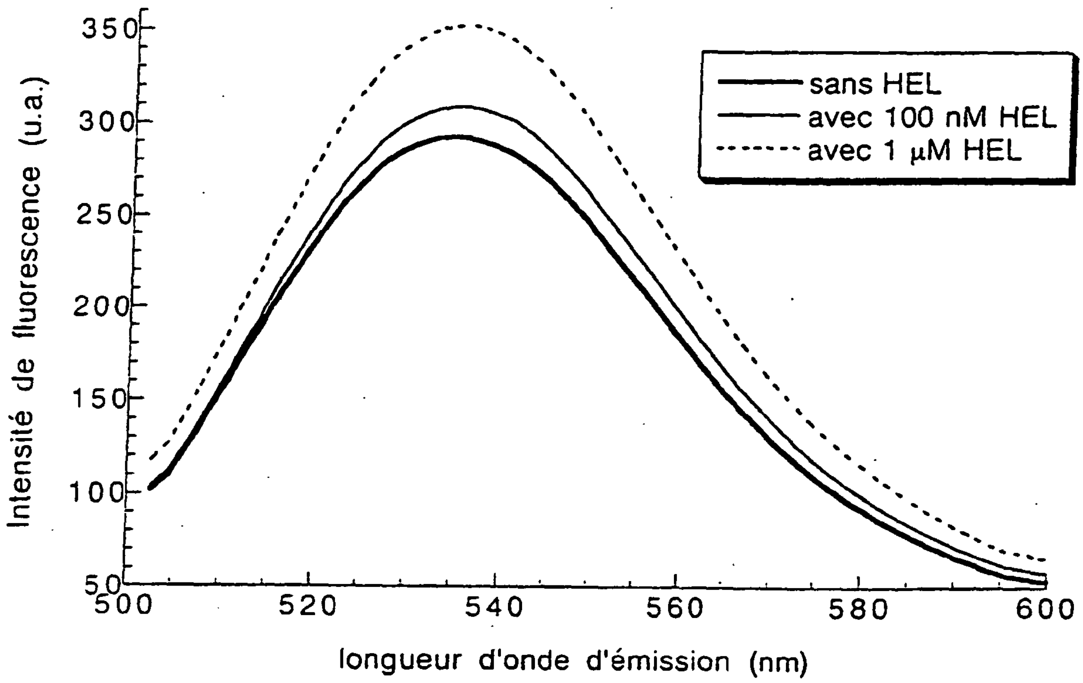
sans HEL
avec 100 nM HEL
avec 1 µM HEL
Intensité de fluorescence (u.a.)
350
300
250
200
150
100
50
500
520
540
560
580
600
longueur d'onde d'émission (nm)

FIGURE 11

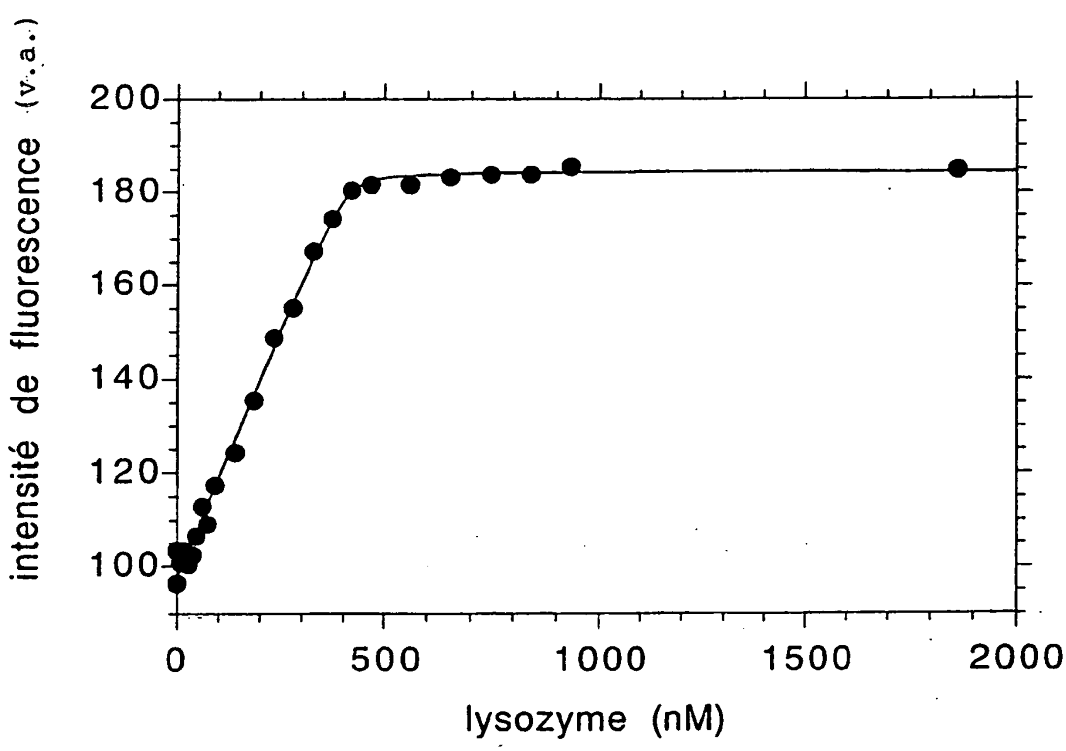
intensité de fluorescence (v.a.)
200
180
160
140
120
100
0
500
1000
1500
2000
lysozyme (nM)

FIGURE 12

| Position | Rendement de couplage (%) | Variation de Fluorescence (%) |
|---|---|---|
| wt | < 8% | |
| VL-His30 | 116% | + 7 % |
| VL-Asn31 | 103% | + 16 % |
| VL-Tyr49 | 78% | + 75 % |
| VL-Tyr50 | 88% | + 1 % |
| VL-Thr52 | 83% | + 12 % |
| VL-Thr53 | 104% | + 13 % |
| VL-Asp56 | 116% | - 1 % |
| VL-Trp92 | 107% | + 53 % |
| VL-Ser93 | 83% | + 26 % |
| VL-Thr94 | 94% | + 55 % |
| VH-Ser28 | 111% | - 2 % |
| VH-Thr30 | 72% | - 6 % |
| VH-Gly31 | 130% | - 2 % |
| VH-Tyr32 | 85% | + 26 % |
| VH-Gly53 | 42% | + 1 % |
| VH-Asn56 | 92% | + 5 % |
| VH-Arg99 | 101% | - 6 % |

FIGURE 13

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9934212 A
- US 5756351 A
- FR 0002657


### Littérature non-brevet citée dans la description


- **Arndt K.M. et al.** *Biochemistry,* 1998, vol. 37, 12918-12926
- **Bhat T.N. et al.** *Proc. Natl. Acad. Sci. USA,* 1994, vol. 91, 1089-1096
- **Braden B.C. et al.** *Immunological Review,* 1998, vol. 163, 45-57
- **Carter P. et al.** *Nucleic Acids Research,* 1985, vol. 13, 4431-4443
- **Creighton T.E.** Proteins: Structure and molecular properties. W.H. Freeman and Cie, 1996, 227-229
- **Dall'Acqua W. et al.** *Biochemistry,* 1996, vol. 35, 9667-9676
- **Dall'Acqua W. et al.** *Current Opinion in Structural Biology,* 1998, vol. 8, 443-450
- **Del Boccio G. et al.** *The Journal of Biochemical Chemistry,* 1991, vol. 266, 13777-13782
- **England P. et al.** *Biochemistry,* 1997, vol. 36, 164-172
- **England P. et al.** *The Journal of Immunology,* 1999, vol. 162, 2129-2136
- **Gilardi G. et al.** *Analytical Chem,* 1994, vol. 66, 3840-3847
- **Glockshuber R. et al.** *Biochemistry,* 1992, vol. 31, 1270-1279
- **Goldbaum FA. et al.** *J. Mol. Recogn.,* 1996, vol. 9, 6-12
- **Haugland R.P.** Handbook of fluorescent probes and research chemicals. Molecular Probes Inc, 1996
- **Hawkins RE. et al.** *J. Mol. Biol.,* 1993, vol. 234, 958-964
- **Houk W.T. et al.** *The Journal of Biological Chemistry,* 1983, vol. 258 (9), 5419-5423
- **Ito W. et al.** *J. Biol. Chem,* 1993, vol. 268, 16639-16647
- **Ito W. et al.** *J. Mol. Biol.,* 1995, vol. 248, 729-732
- **Kunkel T.A et al.** *Methods in Enzymology,* 1987, vol. 154, 367-382
- **Langedijk A.C. et al.** *Journal of Molecular Biology,* 1998, vol. 283, 95-110
- **Marvin J.S. et al.** *Proc. Natl. Acad. Sci. USA,* 1997, vol. 94, 4366-4371
- **Marvin J.S. et al.** *J. Am. Chem. Soc.,* 1998, vol. 120, 7-11
- **Narazaki et al.** *Biochimica et Biophysica Acta,* 1997, vol. 1338, 275-281
- **Pace C.N. et al.** *Protein Structure,* 1995, vol. 4, 2411-2423
- **Piervincenzi RT. et al.** *Biosensors & Bioelectronics,* 1998, vol. 13, 305-312
- **Pollack S.J. et al.** *Science,* 1988, vol. 242, 1038-1040
- **Plückthun A. et al.** Antibody engineering, a practical approach. Oxford University Press, 1996, 203-252
- **Rees A.R. et al.** Protein structure prediction, a practical approach. Oxford University Press, 1996, 141-172
- **Saleemuddin M.** *Adv. Biochem. Eng. Biotech.,* 1999, vol. 64, 203-226
- **Sambrook J. et al.** Molecular cloning: a laboratory manual. Cold Spring Harbor Laboratory Press, 1989
- **Sara M. et al.** *Micron.,* 1996, vol. 27, 141-156
- **Skerra, A.** *Gene,* 1994, vol. 51, 131-135
- **Sloan D.J. et al.** *Protein Engineering,* 1998, vol. 11, 819-23
- **Sternberg M.J.E.** Protein Structure Prediction, a practical approach. Oxford University Press, 1996
- **Studier F.W. ; Moffatt, B.A.** *Journal of Molecular Biology,* 1986, vol. 189, 113-130
- **Tolosa L. et al.** *Analytical Biochem,* 1999, vol. 267, 114-120
- **Turkova J.** *J. Chromatography,* 1999, vol. 722, 11-31
- **Vriends G.** *Journal of Molecular Graphism,* 1990, vol. 8, 52-56
- **Weetall H.H.** *Appl. Biochem. Biotech.,* 1993, vol. 41, 157-188
- **Yoshioka M. et al.** *J. Chromatography,* 1991, vol. 566, 361-368

• **Ysern X. et al.** *J. Mol. Biol,* 1994, vol. 238, 496-500